# EUROPEAN PATENT APPLICATION

(11) **EP 4 786 473 A1**
(43) Date of publication of application: **05.08.2026**
(21) Application number: 24870934.7
(22) Date of filing: 27.09.2024
(51) Int. Cl.: C07D 519/00, C07D 403/14, C07D 487/04, A61K 31/517, A61K 31/439, A61P 35/00

(54) **PHARMACEUTICALLY ACCEPTABLE SALT AND CRYSTALLINE FORM OF QUINAZOLINE DERIVATIVE, AND USE THEREOF**

(30) Priority: 27.09.2023 CN 202311255208
(71) Applicant: Jiangsu Hengrui Pharmaceuticals Co., Ltd., Lianyungang, Jiangsu 222047 (CN); Shanghai Hengrui Pharmaceutical Co., Ltd., Shanghai 200245 (CN)
(72) Inventor: WANG, Jie, Lianyungang, Jiangsu 222047 (CN); YANG, Junran, Lianyungang, Jiangsu 222047 (CN); DU, Zhenxing, Lianyungang, Jiangsu 222047 (CN); WANG, Jie, Lianyungang, Jiangsu 222047 (CN)
(74) Representative: dompatent
(86) International application number: PCT/CN2024/121641
(87) International publication number: WO 2025/067396

(57) **Abstract**

The present disclosure relates to a pharmaceutically acceptable salt and a crystalline form of a quinazoline derivative, and use thereof. Specifically, provided are a pharmaceutically acceptable salt of, a crystal form of, and a preparation method for 1-(endo-3-((4-((4-([1,2,4]triazolo[1,5-a]pyridin-7-yloxy)-2-fluoro-3-methylphenyl)amino)quinazolin-6-yl)oxy)-8-azabicyclo[3.2.1]oct-8-yl)prop-2-en-1-one; the corresponding salt has good stability and can be better used in clinical treatment.

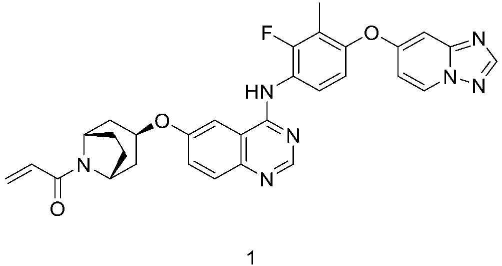

## Description

**The present invention claims the right of priority for:**
Application No.: CN 202311255208.3, filing date: September 27, 2023.

### Technical Field

The present disclosure belongs to the technical field of medicine, and relates to a pharmaceutically acceptable salt and a crystalline form of a quinazoline derivative, and the use thereof.

### Background Art

Human epidermal growth factor receptor 2 (HER2; Neu, ERBB2) is a member of the type I receptor tyrosine kinase family, which also includes EGFR (ERBB1), HER3 (ERBB3) and HER4 (ERBB4). To date, no ligand capable of directly binding to HER2 has been identified in the human body. HER2 activation requires dimerization by forming homo- or heterodimers with other family members, such as HER3. Upon dimerization, the conformation of HER2 undergoes a change, which activates the intracellular tyrosine kinase domain and subsequently activates downstream pathways (MAPK signalling pathway and PI3K/AKT signalling pathway), thereby exerting the corresponding physiological effects.

Statistically, about 2%-4% of lung cancer patients harbour activating mutations in exon 20 of the HER2 gene. Currently clinically approved ERBB-targeted tyrosine kinase inhibitors have negligible efficacy in these patients, primarily due to dose-limiting toxicities mediated by wild-type EGFR. Alectinib, ibrutinib, neratinib, poziotinib, and pyrotinib are known broad-spectrum ERBB inhibitors targeting HER2 exon 20 mutations. Clinically, afatinib and other broad-spectrum ERBB inhibitors have demonstrated only limited efficacy in NSCLC patients with HER2 exon 20 mutations due to the limitation of effective dose.

PCT/CN2023/084265 provides a novel inhibitor targeting the HER2 exon 20 mutations that is selective for the EGFR wild type, wherein the inhibitor has a chemical name of 1-(endo-3-((4-((4-([1,2,4]triazolo[1,5-a]pyridin-7-yloxy)-2-fluoro-3-methylphenyl)amino)quinazolin-6-yl)oxy)-8-azabicyclo[3.2.1]oct-8-yl)prop-2-en-1-one and a structure represented by formula 1

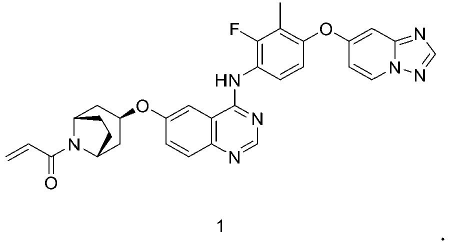

Salt formation can improve some undesirable physical, chemical or biological properties of drugs. It is of great significance to develop salts that are more excellent in physical and chemical properties or pharmaceutical properties than 1-(endo-3-((4-((4-([1,2,4]triazolo[1,5-a]pyridin-7-yloxy)-2-fluoro-3-methylphenyl)amino)quinazolin-6-yl)oxy)-8-azabicyclo[3.2.1]oct-8-yl)prop-2-en-1-one. In view of the importance of solid drug crystal forms and their stability in clinical treatment, it is also of great significance to study the polymorphism of a pharmaceutically acceptable salt of the compound 1-(endo-3-((4-((4-([1,2,4]triazolo[1,5-a]pyridin-7-yloxy)-2-fluoro-3-methylphenyl)amino)quinazolin-6-yl)oxy)-8-azabicyclo[3.2.1]oct-8-yl)prop-2-en-1-one in depth for developing suitable drugs for industrial production with good biological activity.

### Summary of the Invention

The present disclosure provides a pharmaceutically acceptable salt of a compound of formula 1, i.e., 1-(endo-3-((4-((4-([1,2,4]triazolo[1,5-a]pyridin-7-yloxy)-2-fluoro-3-methylphenyl)amino)quinazolin-6-yl)oxy)-8-azabicyclo[3.2.1]oct-8-yl)prop-2-en-1-one, wherein the pharmaceutically acceptable salt is selected from a hydrochloride, a sulfate, a phosphate, a methanesulfonate, a tartrate, a p-toluenesulfonate, and a fumarate,

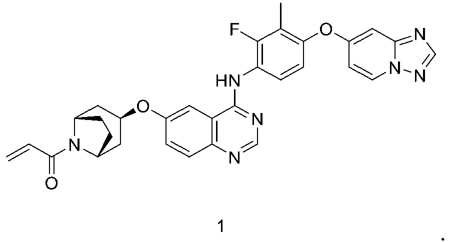

In an optional embodiment, the chemical ratio of the compound of formula 1, i.e., 1-(endo-3-((4-((4-([1,2,4]triazolo[1,5-a]pyridin-7-yloxy)-2-fluoro-3-methylphenyl)amino)quinazolin-6-yl)oxy)-8-azabicyclo[3.2.1]oct-8-yl)prop-2-en-1-one, to an acid is 3 : 1-1 : 3, including but limited to 3 : 1, 2 : 1, 1 : 1, 1 : 2 and 1 : 3.

In another embodiment, the chemical ratio of the compound of formula 1, i.e., 1-(endo-3-((4-((4-([1,2,4]triazolo[1,5-a]pyridin-7-yloxy)-2-fluoro-3-methylphenyl)amino)quinazolin-6-yl)oxy)-8-azabicyclo[3.2.1]oct-8-yl)prop-2-en-1-one, to an acid is 2 : 1-1 : 2.

In an optional embodiment, the chemical ratio of the compound of formula 1, i.e., 1-(endo-3-((4-((4-([1,2,4]triazolo[1,5-a]pyridin-7-yloxy)-2-fluoro-3-methylphenyl)amino)quinazolin-6-yl)oxy)-8-azabicyclo[3.2.1]oct-8-yl)prop-2-en-1-one, to hydrochloric acid is 1 : 1.

In an optional embodiment, the chemical ratio of the compound of formula 1, i.e., 1-(endo-3-((4-((4-([1,2,4]triazolo[1,5-a]pyridin-7-yloxy)-2-fluoro-3-methylphenyl)amino)quinazolin-6-yl)oxy)-8-azabicyclo[3.2.1]oct-8-yl)prop-2-en-1-one, to sulfuric acid is 1 : 1 or 2 : 1.

In an optional embodiment, the chemical ratio of the compound of formula 1, i.e., 1-(endo-3-((4-((4-([1,2,4]triazolo[1,5-a]pyridin-7-yloxy)-2-fluoro-3-methylphenyl)amino)quinazolin-6-yl)oxy)-8-azabicyclo[3.2.1]oct-8-yl)prop-2-en-1-one, to phosphoric acid is 1 : 1 or 1 : 2.

In an optional embodiment, the chemical ratio of the compound of formula 1, i.e., 1-(endo-3-((4-((4-([1,2,4]triazolo[1,5-a]pyridin-7-yloxy)-2-fluoro-3-methylphenyl)amino)quinazolin-6-yl)oxy)-8-azabicyclo[3.2.1]oct-8-yl)prop-2-en-1-one, to methanesulfonic acid is 1 : 1.

In an optional embodiment, the chemical ratio of the compound of formula 1, i.e., 1-(endo-3-((4-((4-([1,2,4]triazolo[1,5-a]pyridin-7-yloxy)-2-fluoro-3-methylphenyl)amino)quinazolin-6-yl)oxy)-8-azabicyclo[3.2.1]oct-8-yl)prop-2-en-1-one, to fumaric acid is 1 : 1 or 2 : 1.

In an optional embodiment, the chemical ratio of the compound of formula 1, i.e., 1-(endo-3-((4-((4-([1,2,4]triazolo[1,5-a]pyridin-7-yloxy)-2-fluoro-3-methylphenyl)amino)quinazolin-6-yl)oxy)-8-azabicyclo[3.2.1]oct-8-yl)prop-2-en-1-one, to p-toluenesulfonic acid is 1 : 1.

In an optional embodiment, the chemical ratio of the compound of formula 1, i.e., 1-(endo-3-((4-((4-([1,2,4]triazolo[1,5-a]pyridin-7-yloxy)-2-fluoro-3-methylphenyl)amino)quinazolin-6-yl)oxy)-8-azabicyclo[3.2.1]oct-8-yl)prop-2-en-1-one, to L-tartaric acid is 1 : 1.

The present disclosure also provides a method for preparing a pharmaceutically acceptable salt of a compound of formula 1, comprising a step of subjecting the compound of formula 1, i.e., 1-(endo-3-((4-((4-([1,2,4]triazolo[1,5-a]pyridin-7-yloxy)-2-fluoro-3-methylphenyl)amino)quinazolin-6-yl)oxy)-8-azabicyclo[3.2.1]oct-8-yl)prop-2-en-1-one, to a reaction with an acid, wherein the acid is selected from hydrochloric acid, sulfuric acid, phosphoric acid, methanesulfonic acid, tartaric acid, p-toluenesulfonic acid, and fumaric acid.

The solvent used for salt formation in the present disclosure is selected from, but not limited to, acetone, tetrahydrofuran, n-propanol, and propylene glycol methyl ether.

The present disclosure provides a crystal form I of a hydrochloride of a compound of formula 1, wherein the crystal form I of the hydrochloride has an X-ray powder diffraction pattern expressed with diffraction angle 2*θ* in which there are characteristic peaks at 8.985, 13.114, 13.459, 17.385, 20.631 and 22.030.

In some embodiments, the crystal form I of the hydrochloride of the compound of formula 1 has an X-ray powder diffraction pattern expressed with diffraction angle 2*θ* in which there are characteristic peaks at 8.985, 9.882, 12.201, 13.114, 13.459, 17.385, 19.936, 20.631 and 22.030.

In some embodiments, the crystal form I of the hydrochloride of the compound of formula 1 has an X-ray powder diffraction pattern expressed with diffraction angle 2*θ* in which there are characteristic peaks at 8.985, 9.882, 12.201, 13.114, 13.459, 14.948, 15.316, 17.385, 19.936, 20.631, 22.030, 24.949, 25.476 and 28.632.

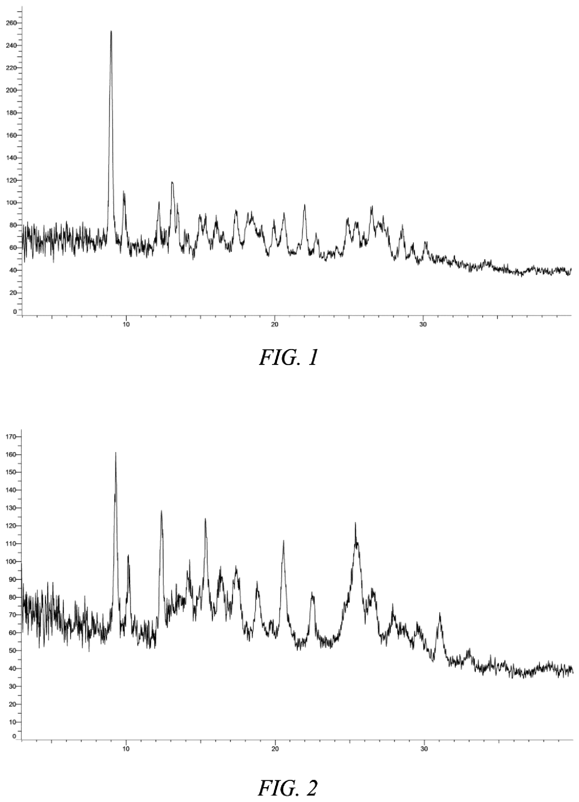

In some embodiments, the crystal form I of the hydrochloride of the compound of formula 1 has an X-ray powder diffraction pattern expressed with diffraction angle 2*θ* as shown in FIG. 1.

The present disclosure also provides a method for preparing a crystal form I of a hydrochloride of a compound of formula 1, comprising dissolving the compound of formula 1 in solvent I, adding an aqueous hydrochloric acid solution, and stirring the mixture, wherein the solvent I is selected from acetone and tetrahydrofuran.

The present disclosure provides a crystal form II of a hydrochloride of a compound of formula 1, wherein the crystal form II of the hydrochloride has an X-ray powder diffraction pattern expressed with diffraction angle 2*θ* in which there are characteristic peaks at 9.315, 12.380, 15.334, 20.554 and 25.413.

In some embodiments, the crystal form II of the hydrochloride of the compound of formula 1 has an X-ray powder diffraction pattern expressed with diffraction angle 2*θ* in which there are characteristic peaks at 9.315, 10.156, 12.380, 15.334, 20.554, 22.482, 25.413 and 31.048.

In some embodiments, the crystal form II of the hydrochloride of the compound of formula 1 has an X-ray powder diffraction pattern expressed with diffraction angle 2*θ* in which there are characteristic peaks at 9.315, 10.156, 12.380, 15.334, 16.379, 17.371, 18.806, 20.554, 22.482, 25.413 and 31.048.

In some embodiments, the crystal form II of the hydrochloride of the compound of formula 1 has an X-ray powder diffraction pattern expressed with diffraction angle 2*θ* as shown in FIG. 2.

The present disclosure also provides a method for preparing a crystal form II of a hydrochloride of a compound of formula 1, comprising:
method I: dissolving the compound of formula 1 in propylene glycol methyl ether, adding an aqueous hydrochloric acid solution, followed by methyl tert-butyl ether, and stirring the mixture;
method II: heating the crystal form I of the hydrochloride of the compound of formula 1 to 120°C.

The present disclosure provides a crystal form III of a hydrochloride of a compound of formula 1, wherein the crystal form III of the hydrochloride has an X-ray powder diffraction pattern expressed with diffraction angle 2*θ* in which there are characteristic peaks at 8.244, 16.610, 20.856, 24.599 and 30.261.

In some embodiments, the crystal form III of the hydrochloride of the compound of formula 1 has an X-ray powder diffraction pattern expressed with diffraction angle 2*θ* in which there are characteristic peaks at 8.244, 9.195, 12.834, 13.615, 16.610, 20.856, 24.599 and 30.261.

In some embodiments, the crystal form III of the hydrochloride of the compound of formula 1 has an X-ray powder diffraction pattern expressed with diffraction angle 2*θ* in which there are characteristic peaks at 8.244, 9.195, 12.834, 13.615, 16.610, 17.173, 17.902, 19.230, 20.856, 24.599, 26.163, 26.471 and 30.261.

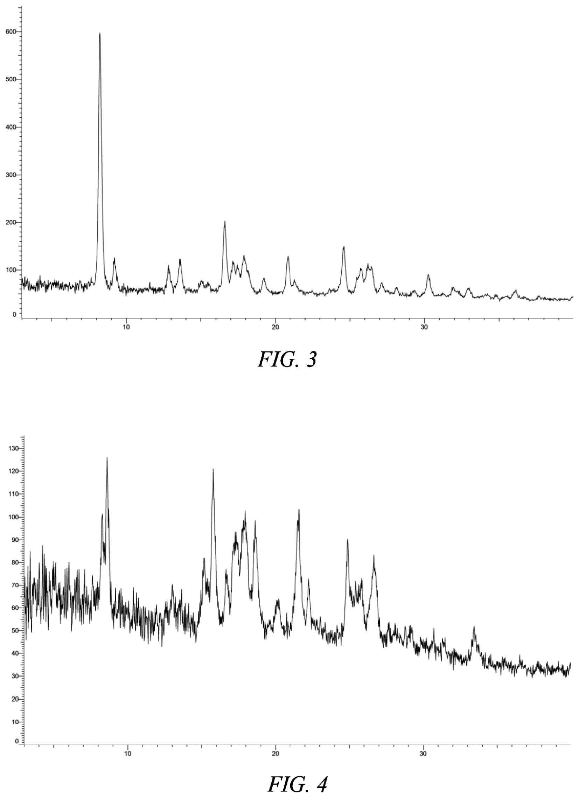

In some embodiments, the crystal form III of the hydrochloride of the compound of formula 1 has an X-ray powder diffraction pattern expressed with diffraction angle 2*θ* as shown in FIG. 3.

The present disclosure also provides a method for preparing a crystal form III of a hydrochloride of a compound of formula 1, comprising adding the compound of formula 1 to an aqueous hydrochloric acid solution, and stirring the mixture.

The present disclosure provides a crystal form I of a sulfate of a compound of formula 1, wherein the crystal form I of the sulfate has an X-ray powder diffraction pattern expressed with diffraction angle 2*θ* in which there are characteristic peaks at 8.249, 8.600, 15.780, 21.562, 24.900 and 26.659.

In some embodiments, the crystal form I of the sulfate of the compound of formula 1 has an X-ray powder diffraction pattern expressed with diffraction angle 2*θ* in which there are characteristic peaks at 8.249, 8.600, 15.191, 15.780, 21.562, 22.265, 24.900, 25.444 and 26.659.

In some embodiments, the crystal form I of the sulfate of the compound of formula 1 has an X-ray powder diffraction pattern expressed with diffraction angle 2*θ* in which there are characteristic peaks at 8.249, 8.600, 15.191, 15.780, 16.723, 17.895, 18.628, 20.099, 21.562, 22.265, 24.900, 25.444 and 26.659.

In some embodiments, the crystal form I of the sulfate of the compound of formula 1 has an X-ray powder diffraction pattern expressed with diffraction angle 2*θ* as shown in FIG. 4.

The present disclosure also provides a method for preparing a crystal form I of a sulfate of a compound of formula 1, comprising dissolving the compound of formula 1 in solvent II, adding an aqueous sulfuric acid solution, and stirring the mixture, wherein the solvent II is selected from acetone, tetrahydrofuran and propylene glycol methyl ether.

The present disclosure provides a crystal form II of a sulfate of a compound of formula 1, wherein the crystal form II of the sulfate has an X-ray powder diffraction pattern expressed with diffraction angle 2*θ* in which there are characteristic peaks at 8.462, 13.537, 18.209, 19.271, 22.193 and 24.694.

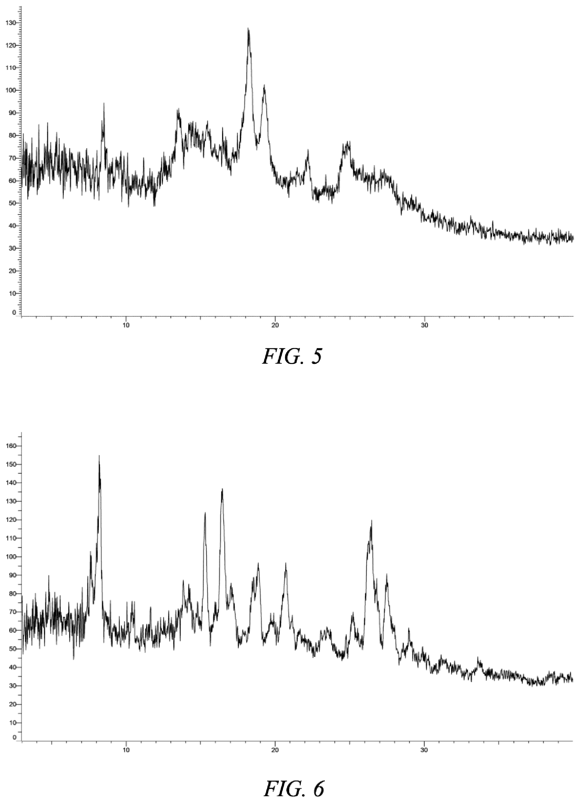

In some embodiments, the crystal form of the compound of formula 1 has an X-ray powder diffraction pattern expressed with diffraction angle 2*θ* as shown in FIG. 5.

The present disclosure also provides a method for preparing a crystal form of a compound of formula 1, comprising heating the crystal form I of the sulfate of the compound of formula 1 to 120°C.

The present disclosure provides a crystal form I of a phosphate of a compound of formula 1, wherein the crystal form I of the phosphate has an X-ray powder diffraction pattern expressed with diffraction angle 2*θ* in which there are characteristic peaks at 8.220, 15.308, 16.443, 18.865, 20.736 and 26.384.

In some embodiments, the crystal form I of the phosphate of the compound of formula 1 has an X-ray powder diffraction pattern expressed with diffraction angle 2*θ* in which there are characteristic peaks at 7.595, 8.220, 15.308, 16.443, 18.536, 18.865, 20.736, 26.384 and 27.495.

In some embodiments, the crystal form I of the phosphate of the compound of formula 1 has an X-ray powder diffraction pattern expressed with diffraction angle 2*θ* in which there are characteristic peaks at 7.595, 8.220, 13.847, 14.222, 15.308, 16.443, 18.536 ,18.865, 20.736, 26.384 and 27.495.

In some embodiments, the crystal form I of the phosphate of the compound of formula 1 has an X-ray powder diffraction pattern expressed with diffraction angle 2*θ* as shown in FIG. 6.

The present disclosure also provides a method for preparing a crystal form I of a phosphate of a compound of formula 1, comprising dissolving the compound of formula 1 in solvent III, adding an aqueous phosphoric acid solution, and stirring the mixture, wherein the solvent III is selected from acetone and tetrahydrofuran.

The present disclosure provides a crystal form II of a phosphate of a compound of formula 1, wherein the crystal form II of the phosphate has an X-ray powder diffraction pattern expressed with diffraction angle 2*θ* in which there are characteristic peaks at 7.659, 14.303, 15.921, 18.824, 20.380 and 26.730.

In some embodiments, the crystal form II of the phosphate of the compound of formula 1 has an X-ray powder diffraction pattern expressed with diffraction angle 2*θ* in which there are characteristic peaks at 7.283, 7.659, 10.049, 14.303, 14.710, 15.921, 17.225, 18.373, 18.824, 19.642, 20.380, 26.357 and 26.730.

In some embodiments, the crystal form II of the phosphate of the compound of formula 1 has an X-ray powder diffraction pattern expressed with diffraction angle 2*θ* in which there are characteristic peaks at 7.283, 7.659, 8.564, 10.049, 12.797, 13.465, 14.303, 14.710, 15.921, 17.225, 18.373, 18.824, 19.642, 20.380, 21.912, 23.628, 24.682, 26.357 and 26.730.

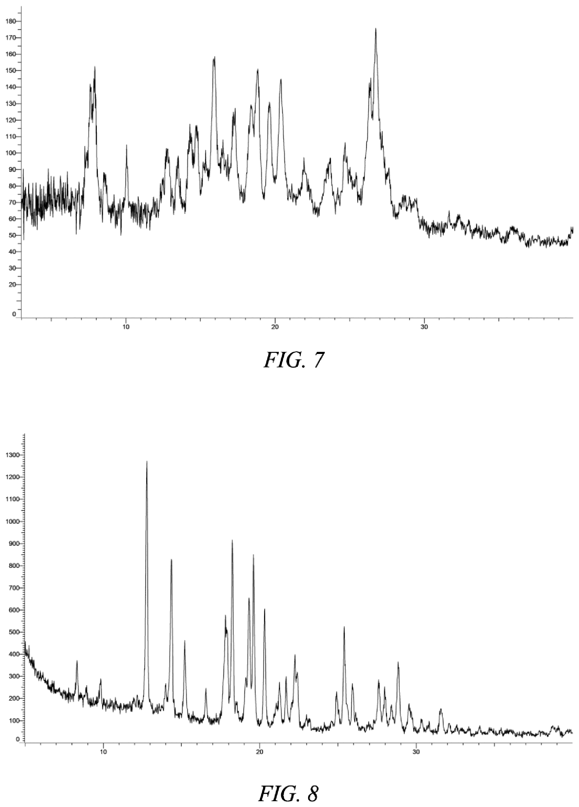

In some embodiments, the crystal form II of the phosphate of the compound of formula 1 has an X-ray powder diffraction pattern expressed with diffraction angle 2*θ* as shown in FIG. 7.

The present disclosure also provides a method for preparing a crystal form II of a phosphate of a compound of formula 1, comprising dissolving the compound of formula 1 in propylene glycol methyl ether, adding an aqueous phosphoric acid solution, and stirring the mixture.

The present disclosure provides a crystal form I of a methanesulfonate of a compound of formula 1, wherein the crystal form I of the methanesulfonate has an X-ray powder diffraction pattern expressed with diffraction angle 2*θ* in which there are characteristic peaks at 12.785, 14.363, 18.248, 19.608, 20.315 and 25.404.

In some embodiments, the crystal form I of the methanesulfonate of the compound of formula 1 has an X-ray powder diffraction pattern expressed with diffraction angle 2*θ* in which there are characteristic peaks at 8.311, 12.785, 14.363, 15.220, 17.817, 18.248, 19.318, 19.608, 20.315, 21.697, 22.256, 25.404 and 28.858.

In some embodiments, the crystal form I of the methanesulfonate of the compound of formula 1 has an X-ray powder diffraction pattern expressed with diffraction angle 2*θ* in which there are characteristic peaks at 8.311, 9.846, 12.785, 14.363, 15.220, 16.576, 17.817, 18.248, 19.318, 19.608, 20.315, 21.697, 22.256, 24.601, 25.404, 27.594, 28.858 and 31.581.

In some embodiments, the crystal form I of the methanesulfonate of the compound of formula 1 has an X-ray powder diffraction pattern expressed with diffraction angle 2*θ* as shown in FIG. 8.

The present disclosure also provides a method for preparing a crystal form I of a methanesulfonate of a compound of formula 1, comprising dissolving the compound of formula 1 in acetone, adding an aqueous methanesulfonic acid solution, and stirring the mixture.

The present disclosure provides a crystal form II of a methanesulfonate of a compound of formula 1, wherein the crystal form II of the methanesulfonate has an X-ray powder diffraction pattern expressed with diffraction angle 2*θ* in which there are characteristic peaks at 8.305, 15.133, 20.915, 23.482 and 25.997.

In some embodiments, the crystal form II of the methanesulfonate of the compound of formula 1 has an X-ray powder diffraction pattern expressed with diffraction angle 2*θ* in which there are characteristic peaks at 8.305, 13.430, 15.133, 16.680, 17.223, 20.915, 21.185, 23.482, 25.428 and 25.997.

In some embodiments, the crystal form II of the methanesulfonate of the compound of formula 1 has an X-ray powder diffraction pattern expressed with diffraction angle 2*θ* in which there are characteristic peaks at 8.305, 9.221, 13.430, 15.133, 16.680, 17.223, 17.943, 19.130, 19.536, 20.915, 21.185, 23.482, 25.428, 25.997, 26.714 and 28.195.

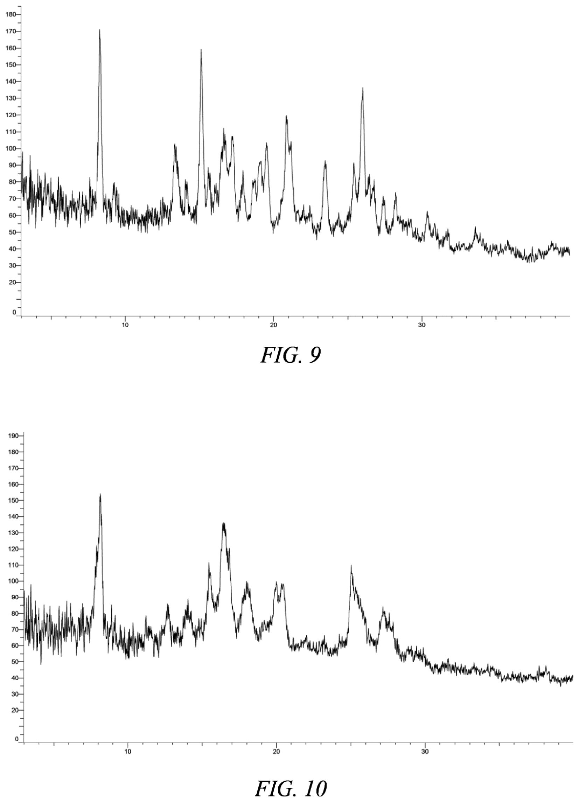

In some embodiments, the crystal form II of the methanesulfonate of the compound of formula 1 has an X-ray powder diffraction pattern expressed with diffraction angle 2*θ* as shown in FIG. 9.

The present disclosure also provides a method for preparing a crystal form II of a methanesulfonate of a compound of formula 1, comprising storing the crystal form I of the methanesulfonate of the compound of formula 1 under conditions of 92.5% RH for 8 days.

The present disclosure provides a crystal form I of a tartrate of a compound of formula 1, wherein the crystal form I of the tartrate has an X-ray powder diffraction pattern expressed with diffraction angle 2*θ* in which there are characteristic peaks at 8.150, 15.441, 16.466, 18.005 and 25.087.

In some embodiments, the crystal form I of the tartrate of the compound of formula 1 has an X-ray powder diffraction pattern expressed with diffraction angle 2*θ* in which there are characteristic peaks at 8.150, 11.190, 12.659, 14.003, 15.441, 16.466, 18.005, 20.010, 20.454, 25.087 and 27.195.

In some embodiments, the crystal form I of the tartrate of the compound of formula 1 has an X-ray powder diffraction pattern expressed with diffraction angle 2*θ* as shown in FIG. 10.

The present disclosure also provides a method for preparing a crystal form I of a tartrate of a compound of formula 1, comprising dissolving the compound of formula 1 in solvent IV, adding an aqueous tartaric acid solution, and stirring the mixture, wherein the solvent IV is selected from one or more of acetone, tetrahydrofuran and propylene glycol methyl ether.

The present disclosure provides a crystal form I of a p-toluenesulfonate of a compound of formula 1, wherein the crystal form I of the p-toluenesulfonate has an X-ray powder diffraction pattern expressed with diffraction angle 2*θ* in which there are characteristic peaks at 6.708, 9.145, 15.275, 17.217, 20.971 and 22.499.

In some embodiments, the crystal form I of the p-toluenesulfonate of the compound of formula 1 has an X-ray powder diffraction pattern expressed with diffraction angle 2*θ* in which there are characteristic peaks at 6.708, 8.935, 9.145, 13.450, 15.275, 16.934, 17.217, 18.123, 18.948, 20.971, 22.499 and 23.831.

In some embodiments, the crystal form I of the p-toluenesulfonate of the compound of formula 1 has an X-ray powder diffraction pattern expressed with diffraction angle 2*θ* in which there are characteristic peaks at 6.579, 6.708, 8.935, 9.145, 12.699, 13.450, 15.275, 16.934, 17.217, 18.123, 18.948, 20.186, 20.971, 22.499, 23.831, 26.139 and 29.589.

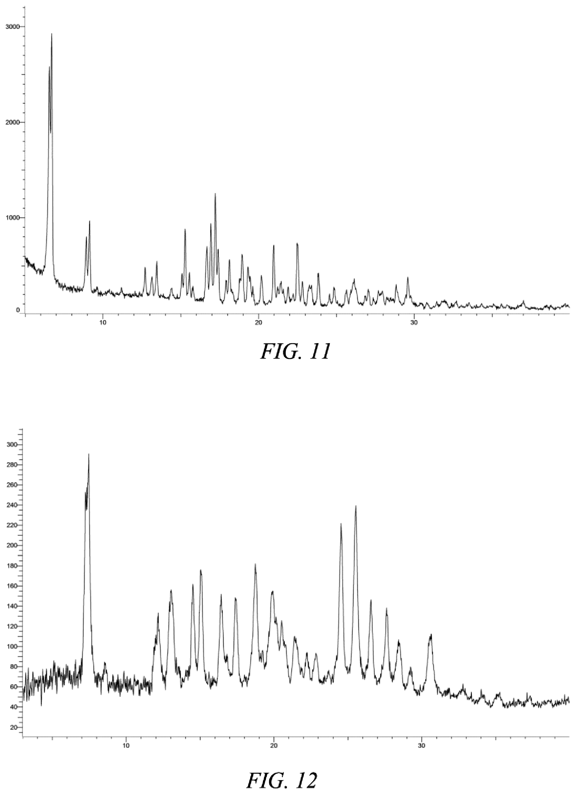

In some embodiments, the crystal form I of the p-toluenesulfonate of the compound of formula 1 has an X-ray powder diffraction pattern expressed with diffraction angle 2*θ* as shown in FIG. 11.

The present disclosure also provides a method for preparing a crystal form I of a p-toluenesulfonate of a compound of formula 1, comprising dissolving the compound of formula 1 in solvent V, adding an aqueous p-toluenesulfonic acid solution, and stirring the mixture, wherein the solvent V is selected from one of acetone, acetone/n-propanol (v/v = 7 : 3), tetrahydrofuran, and acetone/propylene glycol methyl ether (v/v = 1 : 1 or 7 : 3).

The present disclosure provides a crystal form II of a p-toluenesulfonate of a compound of formula 1, wherein the crystal form II of the p-toluenesulfonate has an X-ray powder diffraction pattern expressed with diffraction angle 2*θ* in which there are characteristic peaks at 7.470, 13.033, 15.060, 18.754, 24.552 and 25.528.

In some embodiments, the crystal form II of the p-toluenesulfonate of the compound of formula 1 has an X-ray powder diffraction pattern expressed with diffraction angle 2*θ* in which there are characteristic peaks at 7.470, 12.135, 13.033, 14.508, 15.060, 18.754, 24.552, 25.528, 26.561, 27.634 and 30.575.

In some embodiments, the crystal form II of the p-toluenesulfonate of the compound of formula 1 has an X-ray powder diffraction pattern expressed with diffraction angle 2*θ* in which there are characteristic peaks at 7.470, 12.135, 13.033, 14.508, 15.060, 16.414, 17.415, 18.754, 19.902, 24.552, 25.528, 26.561, 27.634, 28.458, 29.227 and 30.575.

In some embodiments, the crystal form II of the p-toluenesulfonate of the compound of formula 1 has an X-ray powder diffraction pattern expressed with diffraction angle 2*θ* as shown in FIG. 12.

The present disclosure also provides a method for preparing a crystal form II of a p-toluenesulfonate of a compound of formula 1, comprising dissolving the compound of formula 1 in propylene glycol methyl ether, adding an aqueous p-toluenesulfonic acid solution, and stirring the mixture.

The present disclosure provides a crystal form I of a fumarate of a compound of formula 1, wherein the crystal form I has an X-ray powder diffraction pattern expressed with diffraction angle 2*θ* in which there are characteristic peaks at 5.395, 11.651, 11.981, 16.295, 17.798, 19.316 and 22.420.

In some embodiments, the crystal form I of the fumarate of the compound of formula 1 has an X-ray powder diffraction pattern expressed with diffraction angle 2*θ* in which there are characteristic peaks at 5.395, 11.651, 11.981, 13.483, 13.992, 14.973, 16.295, 16.893, 17.798, 19.316, 22.420, 26.663 and 27.471.

In some embodiments, the crystal form I of the fumarate of the compound of formula 1 has an X-ray powder diffraction pattern expressed with diffraction angle 2*θ* in which there are characteristic peaks at 5.395, 11.651, 11.981, 13.483, 13.992, 14.973, 16.295, 16.893, 17.798, 19.316, 22.420, 23.600, 24.100, 25.194, 25.569, 26.663 and 27.471.

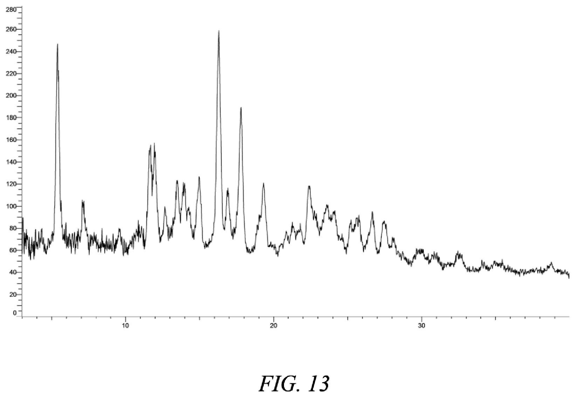

In some embodiments, the crystal form I of the fumarate of the compound of formula 1 has an X-ray powder diffraction pattern expressed with diffraction angle 2*θ* as shown in FIG. 13.

The present disclosure also provides a method for preparing a crystal form I of a fumarate of a compound of formula 1, comprising dissolving the compound of formula 1 in solvent VI, adding solid fumaric acid, and stirring the mixture, wherein the solvent VI is selected from one or more of acetone, tetrahydrofuran and propylene glycol methyl ether.

The present disclosure also provides a pharmaceutical composition, which comprises the above-mentioned pharmaceutically acceptable salt of 1-(endo-3-((4-((4-([1,2,4]triazolo[1,5-a]pyridin-7-yloxy)-2-fluoro-3-methylphenyl)amino)quinazolin-6-yl)oxy)-8-azabicyclo[3.2.1]oct-8-yl)prop-2-en-1-one, or the above-mentioned crystal form I of the hydrochloride, crystal form II of the hydrochloride, crystal form III of the hydrochloride, crystal form I of the sulfate, crystal form II of the sulfate, crystal form I of the phosphate, crystal form II of the phosphate, crystal form I of the methanesulfonate, crystal form II of the methanesulfonate, crystal form I of the tartrate, crystal form I of the p-toluenesulfonate, crystal form II of the p-toluenesulfonate or crystal form I of the fumarate of 1-(endo-3-((4-((4-([1,2,4]triazolo[1,5-a]pyridin-7-yloxy)-2-fluoro-3-methylphenyl)amino)quinazolin-6-yl)oxy)-8-azabicyclo[3.2.1]oct-8-yl)prop-2-en-1-one, and optionally pharmaceutical auxiliaries selected from pharmaceutically acceptable excipients.

The present disclosure also provides a pharmaceutical composition, which is prepared from the above-mentioned pharmaceutically acceptable salt of 1-(endo-3-((4-((4-([1,2,4]triazolo[1,5-a]pyridin-7-yloxy)-2-fluoro-3-methylphenyl)amino)quinazolin-6-yl)oxy)-8-azabicyclo[3.2.1]oct-8-yl)prop-2-en-1-one, or the above-mentioned crystal form I of the hydrochloride, crystal form II of the hydrochloride, crystal form III of the hydrochloride, crystal form I of the sulfate, crystal form II of the sulfate, crystal form I of the phosphate, crystal form II of the phosphate, crystal form I of the methanesulfonate, crystal form II of the methanesulfonate, crystal form I of the tartrate, crystal form I of the p-toluenesulfonate, crystal form II of the p-toluenesulfonate or crystal form I of the fumarate of 1-(endo-3-((4-((4-([1,2,4]triazolo[1,5-a]pyridin-7-yloxy)-2-fluoro-3-methylphenyl)amino)quinazolin-6-yl)oxy)-8-azabicyclo[3.2.1]oct-8-yl)prop-2-en-1-one, and optionally a pharmaceutically acceptable excipient.

The present disclosure also provides a method for preparing a pharmaceutical composition, comprising a step of mixing the above-mentioned pharmaceutically acceptable salt of 1-(endo-3-((4-((4-([1,2,4]triazolo[1,5-a]pyridin-7-yloxy)-2-fluoro-3-methylphenyl)amino)quinazolin-6-yl)oxy)-8-azabicyclo[3.2.1]oct-8-yl)prop-2-en-1-one, or the above-mentioned crystal form I of the hydrochloride, crystal form II of the hydrochloride, crystal form III of the hydrochloride, crystal form I of the sulfate, crystal form II of the sulfate, crystal form I of the phosphate, crystal form II of the phosphate, crystal form I of the methanesulfonate, crystal form II of the methanesulfonate, crystal form I of the tartrate, crystal form I of the p-toluenesulfonate, crystal form II of the p-toluenesulfonate or crystal form I of the fumarate of 1-(endo-3-((4-((4-([1,2,4]triazolo[1,5-a]pyridin-7-yloxy)-2-fluoro-3-methylphenyl)amino)quinazolin-6-yl)oxy)-8-azabicyclo[3.2.1]oct-8-yl)prop-2-en-1-one with a pharmaceutically acceptable excipient.

The present disclosure also provides the use of the above-mentioned pharmaceutically acceptable salt of 1-(endo-3-((4-((4-([1,2,4]triazolo[1,5-a]pyridin-7-yloxy)-2-fluoro-3-methylphenyl)amino)quinazolin-6-yl)oxy)-8-azabicyclo[3.2.1]oct-8-yl)prop-2-en-1-one, or the above-mentioned crystal form I of the hydrochloride, crystal form II of the hydrochloride, crystal form III of the hydrochloride, crystal form I of the sulfate, crystal form II of the sulfate, crystal form I of the phosphate, crystal form II of the phosphate, crystal form I of the methanesulfonate, crystal form II of the methanesulfonate, crystal form I of the tartrate, crystal form I of the p-toluenesulfonate, crystal form II of the p-toluenesulfonate or crystal form I of the fumarate of 1-(endo-3-((4-((4-([1,2,4]triazolo[1,5-a]pyridin-7-yloxy)-2-fluoro-3-methylphenyl)amino)quinazolin-6-yl)oxy)-8-azabicyclo[3.2.1]oct-8-yl)prop-2-en-1-one, or the above-mentioned composition, as a HER2 inhibitor.

The present disclosure also provides the use of the above-mentioned pharmaceutically acceptable salt of 1-(endo-3-((4-((4-([1,2,4]triazolo[1,5-a]pyridin-7-yloxy)-2-fluoro-3-methylphenyl)amino)quinazolin-6-yl)oxy)-8-azabicyclo[3.2.1]oct-8-yl)prop-2-en-1-one, or the above-mentioned crystal form I of the hydrochloride, crystal form II of the hydrochloride, crystal form III of the hydrochloride, crystal form I of the sulfate, crystal form II of the sulfate, crystal form I of the phosphate, crystal form II of the phosphate, crystal form I of the methanesulfonate, crystal form II of the methanesulfonate, crystal form I of the tartrate, crystal form I of the p-toluenesulfonate, crystal form II of the p-toluenesulfonate or crystal form I of the fumarate of 1-(endo-3-((4-((4-([1,2,4]triazolo[1,5-a]pyridin-7-yloxy)-2-fluoro-3-methylphenyl)amino)quinazolin-6-yl)oxy)-8-azabicyclo[3.2.1]oct-8-yl)prop-2-en-1-one, or the above-mentioned composition in the preparation of a medicament as a HER2 inhibitor.

The present disclosure also provides the use of the above-mentioned pharmaceutically acceptable salt of 1-(endo-3-((4-((4-([1,2,4]triazolo[1,5-a]pyridin-7-yloxy)-2-fluoro-3-methylphenyl)amino)quinazolin-6-yl)oxy)-8-azabicyclo[3.2.1]oct-8-yl)prop-2-en-1-one, or the above-mentioned crystal form I of the hydrochloride, crystal form II of the hydrochloride, crystal form III of the hydrochloride, crystal form I of the sulfate, crystal form II of the sulfate, crystal form I of the phosphate, crystal form II of the phosphate, crystal form I of the methanesulfonate, crystal form II of the methanesulfonate, crystal form I of the tartrate, crystal form I of the p-toluenesulfonate, crystal form II of the p-toluenesulfonate or crystal form I of the fumarate of 1-(endo-3-((4-((4-([1,2,4]triazolo[1,5-a]pyridin-7-yloxy)-2-fluoro-3-methylphenyl)amino)quinazolin-6-yl)oxy)-8-azabicyclo[3.2.1]oct-8-yl)prop-2-en-1-one, or the above-mentioned composition in the preparation of a medicament for the treatment and/or prevention of cancer.

According to the use described in the present disclosure, the cancer is selected from breast cancer, ovarian cancer, gastric cancer, colorectal cancer, cervical cancer, endometrial cancer, bladder cancer, brain cancer, epithelial cancer, oesophageal cancer, mesothelioma, nasopharyngeal carcinoma, oral cancer, thyroid cancer, skin cancer, squamous cell carcinoma, synovioma, and hidradenocarcinoma; preferably, the cancer is selected from breast cancer, ovarian cancer, cervical cancer, endometrial cancer, gastric cancer, colorectal cancer and lung cancer.

The "2*θ* or angle 2*θ*" described in the present disclosure refers to a diffraction angle, and *θ* is a Bragg angle in ° or degrees; and the error range of 2*θ* for each characteristic peak is ± 0.20 (including the rounding of numbers with more than 1 decimal place), in particular -0.20, -0.19, -0.18, -0.17, -0.16, -0.15, -0.14, -0.13, - 0.12, -0.11, -0.10, -0.09, -0.08, -0.07, -0.06, -0.05, -0.04, -0.03, -0.02, -0.01, 0.00, 0.01, 0.02, 0.03, 0.04, 0.05, 0.06, 0.07, 0.08, 0.09, 0.10, 0.11, 0.12, 0.13, 0.14, 0.15, 0.16, 0.17, 0.18, 0.19, or 0.20.

If the content of related substances is measured and calculated data, the numerical values in the present disclosure will inevitably have errors to some extent. Generally, ± 10% is within a reasonable error range. There is a certain degree of error variation in the context where it is used. The error variation does not exceed ±10%, and may be ±9%, ±8%, ±7%, ±6%, ±5%, ±4%, ±3%, ±2%, or ±1%, preferably ±5%.

The starting materials used in the method for preparing the crystal forms in the present disclosure can be any form of compounds, and specific forms include, but are not limited to, amorphous form, any crystal form, hydrate, solvate, etc.

The drying temperature in the present disclosure is generally 25°C-100°C, preferably 40°C-70°C. The drying may be drying under ambient pressure or drying under reduced pressure.

The crystallization method described in the present disclosure includes room temperature crystallization, cooling crystallization, solvent-evaporation crystallization, crystal seed-induced crystallization, etc., wherein the temperature for cooling crystallization is below 65°C, preferably from -10°C to 60°C, and stirring may also be performed during crystallization.

The "differential scanning calorimetry or DSC" described in the present disclosure refers to measuring the temperature difference and heat flow difference between a sample and a reference during the process in which the sample is heated or maintained at a constant temperature, so as to characterize all physical and chemical changes related to thermal effects and obtain the phase change information of the sample.

According to the description of hygroscopic characteristics and the definition of hygroscopic weight gain in "9103 Guidelines for Hygroscopicity of Pharmaceuticals", Chinese Pharmacopoeia, Volume IV, 2015 edition, the following are defined:
deliquescence: absorbing enough moisture to form a liquid;
extremely hygroscopic: hygroscopic weight gain not less than 15%;
hygroscopic: hygroscopic weight gain less than 15% and not less than 2%;
slightly hygroscopic: hygroscopic weight gain less than 2% and not less than 0.2%;
no or almost no hygroscopicity: hygroscopic weight gain less than 0.2%.

The "excipient" described in the present disclosure includes, but is not limited to, any adjuvant, carrier, glidant, sweetener, diluent, preservative, dye/colorant, flavouring agent, surfactant, wetting agent, dispersant, suspending agent, stabilizer, isotonic agent, or emulsifier that has been approved by the US Food and Drug Administration to be acceptable for human or livestock use.

### Brief Description of the Drawings

FIG. 1 shows an XRPD pattern of the crystal form I of the hydrochloride of compound 1.
FIG. 2 shows an XRPD pattern of the crystal form II of the hydrochloride of compound 1.
FIG. 3 shows an XRPD pattern of the crystal form III of the hydrochloride of compound 1.
FIG. 4 shows an XRPD pattern of the crystal form I of the sulfate of compound 1.
FIG. 5 shows an XRPD pattern of the crystal form II of the sulfate of compound 1.
FIG. 6 shows an XRPD pattern of the crystal form I of the phosphate of compound 1.
FIG. 7 shows an XRPD pattern of the crystal form II of the phosphate of compound 1.
FIG. 8 shows an XRPD pattern of the crystal form I of the methanesulfonate of compound 1.
FIG. 9 shows an XRPD pattern of the crystal form II of the methanesulfonate of compound 1.
FIG. 10 shows an XRPD pattern of the crystal form I of the tartrate of compound 1.
FIG. 11 shows an XRPD pattern of the crystal form I of the p-toluenesulfonate of compound 1.
FIG. 12 shows an XRPD pattern of the crystal form II of the p-toluenesulfonate of compound 1.
FIG. 13 shows an XRPD pattern of the crystal form I of the fumarate of compound 1.

### Detailed Description of Embodiments

The present disclosure will be explained in more detail below in conjunction with examples or experimental examples. The examples or experimental examples in the present disclosure are only used to illustrate the technical solutions of the present disclosure and do not limit the essence and scope of the present disclosure.

### Test conditions for instruments used in the experiment:

The structures of the compounds are determined by nuclear magnetic resonance (NMR) or/and mass spectrometry (MS). NMR shifts (δ) are given in a unit of 10-6 (ppm). NMR is determined by Bruker AVANCE-400 nuclear magnetic resonance spectrometer or Bruker AVANCE NEO 500M; the solvents for determination are deuterated dimethyl sulfoxide (DMSO-d6), deuterated chloroform (CDCl3) and deuterated methanol (CD3OD); and the internal standard is tetramethylsilane (TMS).

MS determination is carried out by Agilent 1200 /1290 DAD- 6110/6120 Quadrupole MS liquid chromatography-mass spectrometer (manufacturer: Agilent, MS model: 6110/6120 Quadrupole MS);
waters ACQuity UPLC-QD/SQD (manufacturer: waters, MS model: waters ACQuity Qda Detec-tor/waters SQ Detector); and
THERMO Ultimate 3000-Q Exactive (manufacturer: THERMO, MS model: THERMO Q Exactive).

High-performance liquid chromatography (HPLC) analysis is carried out by Agilent HPLC 1200DAD, Agilent HPLC 1200VWD and Waters HPLC e2695-2489 high-pressure liquid chromatographs.

Chiral HPLC analysis is determined by Agilent 1260 DAD high-performance liquid chromatograph.

Preparative high-performance liquid chromatography is carried out by Waters 2545-2767, Waters 2767-SQ Detecor2, Shimadzu LC-20AP and Gilson GX-281 preparative chromatographs.

Chiral preparative chromatography is carried out by Shimadzu LC-20AP preparative chromatograph.

The CombiFlash flash preparative instrument used is Combiflash Rf200 (TELEDYNE ISCO).

Yantai Huanghai HSGF254 or Qingdao GF254 silica gel plate is used as a thin layer chromatography silica plate, and the silica gel plate for the thin layer chromatography (TLC) is of the specification of 0.15 mm - 0.2 mm, and the specification when separating and purifying a product by thin layer chromatography is 0.4 mm - 0.5 mm.

For silica gel column chromatography, Yantai Huanghai silica gel 200-300 mesh silica gel is generally used as a carrier.

The average kinase inhibition rate and IC50 value are determined using NovoStar microplate reader (BMG Company, Germany).

Known starting materials of the present disclosure may be synthesized using or according to methods known in the art, or may be purchased from ABCR GmbH & Co. KG, Acros Organics, Aldrich Chemical Company, Accela ChemBio Inc., Chembee Chemicals, and other companies.

If there is no special instruction in the examples, reactions can all be carried out under argon atmosphere or nitrogen atmosphere.

The argon atmosphere or nitrogen atmosphere means that the reaction bottle is connected to an argon or nitrogen balloon with a volume of about 1 L.

The hydrogen atmosphere means that the reaction bottle is connected to a hydrogen balloon with a volume of about 1 L.

Pressurized hydrogenation reactions are performed using Parr 3916EKX hydrogenator, Qinglan QL-500 hydrogen generator or HC2-SS hydrogenator.

The hydrogenation reaction usually comprises the steps of vacuumizing and charging with hydrogen, and the operation is repeated 3 times.

CEM Discover-S 908860 microwave reactor is used for the microwave reaction.

Unless otherwise specified in the examples, a solution refers to an aqueous solution.

Unless otherwise specified in the examples, the reaction temperature refers to room temperature, which is 20°C to 30°C.

The progress of the reaction in the examples is monitored by means of thin layer chromatography (TLC). The developing solvent used in the reaction, the eluent system of column chromatography used in the purification of compounds and the developing solvent system of thin layer chromatography include: A: dichloromethane/methanol system, B: n-hexane/ethyl acetate system, C: petroleum ether/ethyl acetate system, D: ethyl acetate/methanol system. The volume ratio of the solvents is adjusted according to the polarity of compounds, and can also be adjusted by adding a small amount of basic reagents such as triethylamine or acidic reagents such as acetic acid.

XRPD refers to X-ray powder diffraction and is determined using BRUKER D8 X-ray Diffractometer, with specific collecting information as follows: Cu anode (40 kV, 40 mA), ray: monochromatic Cu-Ka ray (l = 1.5418Å). Scanning mode: *θ*/2*θ*, scanning range: 3-48°.

DSC refers to differential scanning calorimetry and is determined using METTLER TOLEDO DSC 3+ Differential Scanning Calorimeter, with the heating rate of 10°C/min, wherein the specific temperature range is determined with reference to the corresponding diagram (mostly 25°C to 300°C or 25°C to 350°C), and the nitrogen purge rate of 50 mL/min.

TGA refers to thermogravimetric analysis and is determined using METTLER TOLEDO TGA 2 Thermogravimetric Analyzer, with the heating rate of 10°C/min, wherein the specific temperature range is determined with reference to the corresponding diagram (mostly 30°C to 400°C), and the nitrogen purge rate of 50 mL/min.

DVS refers to dynamic vapor sorption and is determined using SMS DVS Advantage at 25°C, with the humidity change of 50%-95%-0%-95%-50% and the stepping of 10% (5% for the last step) (the specific humidity range is determined in accordance with the corresponding plot, and the methods listed herein are those most commonly used), and the standard for determination is as follow: dm/dt is not more than 0.002%.

Ion chromatography detection: instrument: DIONEX AQUION ion chromatograph (USA), detection method: conductivity; separation column: DionexIonPac AS27-4 × 250 mm, 4 µm; eluent: EGC 500 KOH 35 mM; flow rate: 1.5 mL/min.

### Example 1 Preparation of compound of formula 1

### 1-(endo-3-((4-((4-([1,2,4]triazolo[1,5-a]pyridin-7-yloxy)-2-fluoro-3-methylphenyl)amino)quinazolin-6-yl)oxy)-8-azabicyclo[3.2.1]oct-8-yl)prop-2-en-1-one 1

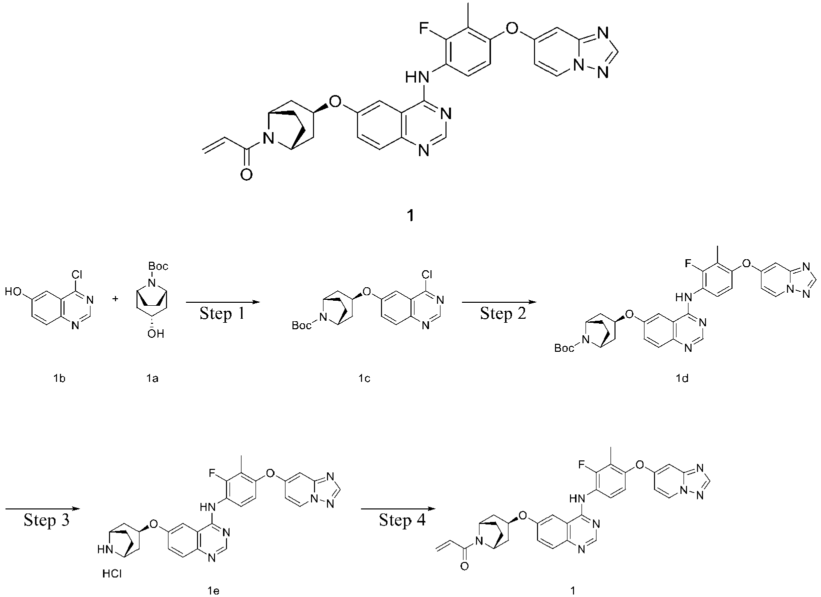

### Step 1

### Tert-butyl endo-3-((4-chloroquinazolin-6-yl)oxy)-8-azabicyclo[3.2.1]octane-8-carboxylate 1c

4-Chloroquinazolin-6-ol **1b** (20 mg, 110 µmol, prepared according to the method disclosed in *"*European Journal of Medicinal Chemistry, 2018, vol. 147, p. 130 - 149"), compound **1a** (30 mg,133 µmol) and triphenylphosphine (58 mg, 221 µmol) were dissolved in tetrahydrofuran (5 mL), and diisopropyl azodicarboxylate (34 mg, 166 µmol) was added dropwise under an ice bath. The mixture was naturally warmed to room temperature, and then reacted for 16 hours. The reaction solution was concentrated under reduced pressure, and then the residue was purified by silica gel column chromatography with eluent system C to obtain title compound **1c** (40 mg, yield: 92.6%). MS m/z (ESI): 390.2 [M+1].

### Step 2

### Tert-butyl endo-3-((4-((4-([1,2,4]triazolo[1,5-a]pyridin-7-yloxy)-2-fluoro-3-methylphenyl)amino)quinazolin-6-yl)oxy)-8-azabicyclo[3.2.1]octane-8-carboxylate 1d

Compound **1c** (1.2 g, 3 mmol) was dissolved in isopropanol (5 mL), and 4-([1,2,4]triazolo[1,5-*a*]pyridin-7-yloxy)-2-fluoro-3-methylaniline (845 mg, 3.27 mmol, prepared according to the method disclosed in patent application "WO 2022003575A1", description, page 143, Example 95) was added. The mixture was reacted at 80°C for 2 hours. The reaction solution was concentrated under reduced pressure. The residue was purified by silica gel column chromatography with eluent system A to obtain title compound **1d** (1.05 g, yield: 55.7%). MS m/z (ESI): 612.2 [M+1].

### Step 3

### 6-((endo-8-azabicyclo[3.2.1]oct-3-yl)oxy)-N-(4-([1,2,4]triazolo[1,5-a]pyridin-7-yloxy)-2-fluoro-3-methylphenyl)quinazolin-4-amine hydrochloride 1e

Compound **1d** (1.05 g, 1.7 mmol) was dissolved in methanol (10 mL), and a solution of hydrogen chloride in dioxane (0.1 mL, 4 M) was added. The mixture was stirred and reacted for 1 hour. The reaction solution was concentrated under reduced pressure to obtain title compound **1e** (crude, 940 mg), which was directly used in the next reaction without purification. MS m/z (ESI): 512.2 [M+1].

### Step 4

### 1-(endo-3-((4-((4-([1,2,4]triazolo[1,5-a]pyridin-7-yloxy)-2-fluoro-3-methylphenyl)amino)quinazolin-6-yl)oxy)-8-azabicyclo[3.2.1]oct-8-yl)prop-2-en-1-one 1

Crude compound **1e** (250 mg, 488.7 µmol) was dissolved in 5 mL of dichloromethane, and *N*,*N*-diisopropylethylamine (126 mg, 974 µmol) was added. Acryloyl chloride (50 mg, 552.4 µmol) was added under an ice bath. The mixture was stirred and reacted for 1 hour. The reaction solution was concentrated under reduced pressure, and the residue was purified by preparative high-performance liquid chromatography (Waters-2545, chromatographic column: YMC Triart-Exrs C18, 30*150 mm, 5 µm; mobile phase: aqueous phase (10 mmol/L ammonium bicarbonate) and acetonitrile, gradient: acetonitrile 30%-45%, flow rate: 30 mL/min) to obtain title compound 1 (110 mg, yield: 39.7%). MS m/z (ESI): 566.2 [M+1]. ¹H NMR (500 MHz, MeOD): δ 8.80-8.78 (d, 1H), 8.41 (s, 1H), 8.33 (s, 1H), 7.84-7.82 (m, 1H), 7.77 (s, 1H), 7.60-7.56 (m, 2H), 7.14-7.10 (m, 2H), 7.02-7.01 (m, 1H), 6.79-6.74 (m, 1H), 6.35 (dd, 1H), 5.81 (dd, 1H), 4.97-4.93 (m, 1H), 4.76-4.73 (m, 1H), 4.61-4.59 (m, 1H), 2.44-2.17 (m, 10H), 2.06-2.02 (m, 1H).

### Test example 1 Ba/F3 cell proliferation assay

EGFR wild-type Ba/F3 cells in the logarithmic growth phase (Cobioer, Cat. No.: CBP73110) were seeded into 96-well plates at a density of 2.5 × 103 cells/100 uL of growth culture medium. HER2 wild-type Ba/F3 cells (Cobioer, Cat. No.: CBP73110) or HER2 A775_G776insYVMA mutant Ba/F3 cells (Cobioer, Cat. No.: CBP73184) in the logarithmic growth phase were seeded into 96-well plates at a density of 5 × 103 cells/100 uL of growth culture medium. The plates were incubated overnight in a 37°C cell incubator. The following day, the compound, which had been serially diluted 3-fold in growth culture medium, was added at 100 uL/well. All treatments were performed in triplicate. The plates were incubated in the 37°C cell incubator for another 72 hours. Celltiter-Glo Luminescent Cell Viability Assay was performed.

The cell proliferation rate was calculated for each well according to the following formula: Proliferation (%) = (G3 - G0 mean value of test compound wells)/(G3 mean value - GO mean value of DMSO control wells)*100. Based on the proliferation rate corresponding to each gradient concentration well and concentration thereof, the gradient curve of cell proliferation was fitted using Prism Graphpad software, and the GI50 of the compound was calculated (GI50 is defined as the compound concentration required to achieve a 50% inhibition rate of cell proliferation).

Tucatinib, a HER2-selective inhibitor (synthesized with reference to Example 11 in patent WO 2007059257 A2), has the following structure:

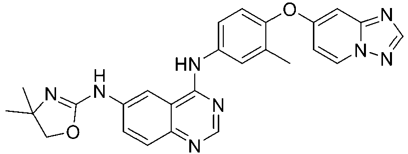

**Table 1**

| Example No. | HER2 WT/Ba F3 GI₅₀ (nM) | HER2 WT/BaF 3 Imax % | HER2 A775_G776 ins YVMA/BaF 3 GI₅₀ (nM) | HER2 A775_G77 6 ins YVMA/Ba F3 Imax % | EGFR WT/BaF3 GI₅₀ (nM) | EGFR WT/BaF 3 Imax % |
|---|---|---|---|---|---|---|
| 1 | 9.3 | 100 | 10.3 | 100 | 2840 | 100 |
| Tucatinib | - | - | 309.23 | 100 | - | - |

Conclusions: the compound of the present disclosure has strong inhibitory effects on the proliferation of HER2 exon 20 YVMA insertion mutation and HER2 wild-type dependent Ba/F3 cells, and exhibits high selectivity relative to EGFR wild-type dependent Ba/F3 cells; furthermore, the inhibitory activity of the compound of the present disclosure on the proliferation of HER2 exon 20 YVMA insertion mutation dependent Ba/F3 cells is significantly superior to that of tucatinib.

### Example 2 Preparation of crystal form I of hydrochloride

8.5 mg of the compound of formula 1 was dissolved in 0.2 mL of acetone to give a clear solution. An aqueous hydrochloric acid solution (8.25 µL, 2 M) was added to the resulting solution. The mixture was stirred and centrifuged. The resulting solid was collected and dried under vacuum to obtain a product. Upon detection by X-ray powder diffraction, the product was defined as crystal form I of the hydrochloride, the XRPD pattern was as shown in FIG. 1, and the positions of the characteristic peaks thereof were as listed in Table 2. The chloride ion content of the product was detected to be 5.58% by ion chromatography. The DSC pattern shows that the peak temperatures of the endothermic peaks were 60.82°C, 94.14°C and 196.79°C. The TGA pattern shows that the weight loss at 30°C-120°C was 3.50%, and the weight loss at 120°C-220°C was 2.02%.

**Table 2**

| Peak number | 2θ value [° or degrees] | d[Å] | Relative intensity % |
|---|---|---|---|
| 1 | 8.985 | 9.83403 | 100.0 |
| 2 | 9.882 | 8.94310 | 19.9 |
| 3 | 12.201 | 7.24821 | 20.8 |
| 4 | 13.114 | 6.74562 | 30.2 |
| 5 | 13.459 | 6.57365 | 17.5 |
| 6 | 13.941 | 6.34739 | 9.3 |
| 7 | 14.948 | 5.92204 | 16.1 |
| 8 | 15.316 | 5.78034 | 10.8 |
| 9 | 16.035 | 5.52275 | 9.2 |
| 10 | 17.385 | 5.09687 | 15.5 |
| 11 | 18.161 | 4.88084 | 12.3 |
| 12 | 18.505 | 4.79092 | 12.2 |
| 13 | 19.099 | 4.64325 | 8.0 |
| 14 | 19.936 | 4.45014 | 12.4 |
| 15 | 20.631 | 4.30170 | 15.8 |
| 16 | 22.030 | 4.03160 | 19.6 |
| 17 | 22.850 | 3.88876 | 5.8 |
| 18 | 24.949 | 3.56614 | 13.6 |
| 19 | 25.476 | 3.49358 | 9.6 |
| 20 | 26.569 | 3.35227 | 17.7 |
| 21 | 27.289 | 3.26545 | 12.9 |
| 22 | 28.632 | 3.11525 | 11.5 |
| 23 | 29.289 | 3.04679 | 2.2 |
| 24 | 30.133 | 2.96335 | 6.7 |

### Example 3: Preparation of crystal form II of hydrochloride

8.5 mg of the compound of formula 1 was dissolved in 0.2 mL of propylene glycol methyl ether to give a clear solution. An aqueous hydrochloric acid solution (8.25 µL, 2 M) and 0.2 ml of methyl tert-butyl ether were added to the resulting solution. The mixture was subjected to dilution crystallization and then centrifuged. The resulting solid was collected and dried under vacuum to obtain a product. Upon detection by X-ray powder diffraction, the product was defined as crystal form II of the hydrochloride, the XRPD pattern was as shown in FIG. 2, and the positions of the characteristic peaks thereof were as listed in Table 3. The chloride ion content of the product was detected to be 6.27% by ion chromatography. The DSC pattern shows that the peak temperatures of the endothermic peaks were 50.83°C and 213.48°C. The TGA pattern shows that the weight loss at 30°C-240°C was 8.88%.

**Table 3**

| Peak number | 2θ value [° or degrees] | d[Å] | Relative intensity % |
|---|---|---|---|
| 1 | 9.315 | 9.48642 | 100.0 |
| 2 | 10.156 | 8.70237 | 44.3 |
| 3 | 12.380 | 7.14367 | 70.6 |
| 4 | 14.128 | 6.26354 | 21.7 |
| 5 | 15.334 | 5.77378 | 52.5 |
| 6 | 16.379 | 5.40757 | 20.5 |
| 7 | 17.371 | 5.10104 | 30.1 |
| 8 | 18.806 | 4.71481 | 23.3 |
| 9 | 20.554 | 4.31764 | 52.5 |
| 10 | 22.482 | 3.95156 | 25.6 |
| 11 | 25.413 | 3.50203 | 59.1 |
| 12 | 26.570 | 3.35215 | 22.3 |
| 13 | 27.914 | 3.19371 | 11.4 |
| 14 | 29.477 | 3.02783 | 3.5 |
| 15 | 31.048 | 2.87812 | 21.3 |

### Example 4: Preparation of crystal form II of hydrochloride

The crystal form I of the hydrochloride of the compound of formula 1 was heated to 120°C to obtain a product. The product was detected to be the crystal form II of the hydrochloride by X-ray powder diffraction.

### Example 5: Preparation of crystal form III of hydrochloride

To 20 mg of the compound of formula 1 was added an aqueous hydrochloric acid solution (2 mL, pH 1.0). The mixture was stirred and centrifuged. The resulting solid was collected and dried under vacuum to obtain a product. Upon detection by X-ray powder diffraction, the product was defined as crystal form III of the hydrochloride, the XRPD pattern was as shown in FIG. 3, and the positions of the characteristic peaks thereof were as listed in Table 4. The chloride ion content of the product was detected to be 5.40% by ion chromatography. The DSC pattern shows that the peak temperatures of the endothermic peaks were 76.96°C, 94.62°C and 196.79°C. The TGA pattern shows that the weight loss at 30°C-120°C was 7.40%, and the weight loss at 120°C-220°C was 2.20%.

**Table 4**

| Peak number | 2θ value [° or degrees] | d[Å] | Relative intensity % |
|---|---|---|---|
| 1 | 8.244 | 10.71669 | 100.0 |
| 2 | 9.195 | 9.60976 | 10.8 |
| 3 | 12.834 | 6.89222 | 9.4 |
| 4 | 13.615 | 6.49844 | 9.8 |
| 5 | 16.610 | 5.33298 | 25.4 |
| 6 | 17.173 | 5.15934 | 9.4 |
| 7 | 17.442 | 5.08038 | 9.1 |
| 8 | 17.902 | 4.95074 | 13.2 |
| 9 | 19.230 | 4.61187 | 5.1 |
| 10 | 20.856 | 4.25586 | 13.4 |
| 11 | 24.599 | 3.61608 | 17.1 |
| 12 | 25.742 | 3.45800 | 7.5 |
| 13 | 26.163 | 3.40329 | 7.3 |
| 14 | 26.471 | 3.36443 | 8.5 |
| 15 | 30.261 | 2.95114 | 7.4 |

### Example 6 Preparation of crystal form I of sulfate

8.5 mg of the compound of formula 1 was dissolved in 0.2 mL of acetone to give a clear solution. An aqueous sulfuric acid solution (8.25 µL, 2 M) was added to the resulting solution. The mixture was stirred and centrifuged. The resulting solid was collected and dried under vacuum to obtain a product. Upon detection by X-ray powder diffraction, the product was defined as crystal form I of the sulfate, the XRPD pattern was as shown in FIG. 4, and the positions of the characteristic peaks thereof were as listed in Table 5. The sulfate ion content of the product was detected to be 15.74% by ion chromatography. The DSC pattern shows that the peak temperatures of the endothermic peaks were 110.48°C and 213.29°C. The TGA pattern shows that the weight loss at 30°C-160°C was 2.40%.

**Table 5**

| Peak number | 2θ value [° or degrees] | d[Å] | Relative intensity % |
|---|---|---|---|
| 1 | 8.249 | 10.71050 | 58.8 |
| 2 | 8.600 | 10.27328 | 100.0 |
| 3 | 12.972 | 6.81930 | 19.9 |
| 4 | 13.597 | 6.50712 | 15.7 |
| 5 | 15.191 | 5.82763 | 40.3 |
| 6 | 15.780 | 5.61160 | 96.1 |
| 7 | 16.723 | 5.29715 | 24.1 |
| 8 | 17.300 | 5.12163 | 42.5 |
| 9 | 17.895 | 4.95269 | 55.6 |
| 10 | 18.628 | 4.75951 | 59.6 |
| 11 | 20.099 | 4.41436 | 11.1 |
| 12 | 21.562 | 4.11804 | 65.4 |
| 13 | 22.265 | 3.98960 | 27.6 |
| 14 | 24.900 | 3.57305 | 58.7 |
| 15 | 25.444 | 3.49780 | 25.0 |
| 16 | 25.851 | 3.44373 | 25.9 |
| 17 | 26.659 | 3.34113 | 48.1 |
| 18 | 33.416 | 2.67939 | 15.8 |

### Example 7: Preparation of crystal form I of sulfate

8.5 mg of the compound of formula 1 was dissolved in 0.2 mL of a solvent as shown in Table 6 below to give a clear solution. An aqueous sulfuric acid solution (8.25 µL, 2 M) was added to the resulting solution. The mixture was stirred and centrifuged. The resulting solid was collected and dried under vacuum to obtain a product. The product was detected to be the crystal form I of the sulfate by X-ray powder diffraction.

**Table 6**

| Solvent | Crystal form |
|---|---|
| Tetrahydrofuran | Crystal form I of sulfate |
| Propylene glycol methyl ether | Crystal form I of sulfate |

### Example 8: Preparation of crystal form II of sulfate

The crystal form I of the sulfate of the compound of formula 1 was heated to 120°C to obtain a product. Upon detection by X-ray powder diffraction, the product was defined as crystal form II of the sulfate, the XRPD pattern was as shown in FIG. 5, and the positions of the characteristic peaks thereof were as listed in Table 7.

**Table 7**

| Peak number | 2θ value [° or degrees] | d[Å] | Relative intensity % |
|---|---|---|---|
| 1 | 8.462 | 10.44097 | 44.6 |
| 2 | 9.658 | 9.15011 | 32.4 |
| 3 | 13.537 | 6.53585 | 50.1 |
| 4 | 14.347 | 6.16850 | 20.7 |
| 5 | 15.410 | 5.74537 | 28.4 |
| 6 | 18.209 | 4.86809 | 100.0 |
| 7 | 19.271 | 4.60219 | 64.4 |
| 8 | 22.193 | 4.00228 | 22.0 |
| 9 | 24.694 | 3.60234 | 31.7 |

### Example 9: Preparation of crystal form I of phosphate

8.5 mg of the compound of formula 1 was dissolved in 0.2 mL of acetone to give a clear solution. An aqueous phosphoric acid solution (8.25 µL, 2 M) was added to the resulting solution. The mixture was stirred and centrifuged. The resulting solid was collected and dried under vacuum to obtain a product. Upon detection by X-ray powder diffraction, the product was defined as crystal form I of the phosphate, the XRPD pattern was as shown in FIG. 6, and the positions of the characteristic peaks thereof were as listed in Table 8. The phosphate ion content of the product was detected to be 17.07% by ion chromatography. The DSC pattern shows that the peak temperatures of the endothermic peaks were 101.48°C, 127.48°C, 192.14°C and 200.30°C. The TGA pattern shows that the weight loss at 30°C-160°C was 1.69%.

**Table 8**

| Peak number | 2θ value [° or degrees] | d[Å] | Relative intensity % |
|---|---|---|---|
| 1 | 7.595 | 11.63037 | 47.7 |
| 2 | 8.220 | 10.74718 | 100.0 |
| 3 | 10.377 | 8.51772 | 16.5 |
| 4 | 11.628 | 7.60442 | 15.4 |
| 5 | 13.847 | 6.39016 | 32.6 |
| 6 | 14.222 | 6.22245 | 27.1 |
| 7 | 14.785 | 5.98689 | 9.5 |
| 8 | 15.308 | 5.78346 | 69.2 |
| 9 | 16.443 | 5.38676 | 84.3 |
| 10 | 17.001 | 5.21113 | 27.5 |
| 11 | 18.536 | 4.78291 | 30.6 |
| 12 | 18.865 | 4.70033 | 39.8 |
| 13 | 19.724 | 4.49746 | 4.8 |
| 14 | 20.736 | 4.28025 | 38.1 |
| 15 | 21.131 | 4.20113 | 9.0 |
| 16 | 23.475 | 3.78659 | 9.5 |
| 17 | 25.163 | 3.53627 | 12.8 |
| 18 | 26.260 | 3.39100 | 59.9 |
| 19 | 26.384 | 3.37530 | 67.5 |
| 20 | 26.788 | 3.32527 | 34.9 |
| 21 | 27.495 | 3.24142 | 38.3 |
| 22 | 28.977 | 3.07894 | 13.3 |

### Example 10: Preparation of crystal form I of phosphate

8.5 mg of the compound of formula 1 was dissolved in 0.2 mL of tetrahydrofuran to give a clear solution. An aqueous phosphoric acid solution (8.25 µL, 2 M) was added to the resulting solution. The mixture was stirred and centrifuged. The resulting solid was collected and dried under vacuum to obtain a product. The product was detected to be the crystal form I of the phosphate by X-ray powder diffraction.

### Example 11: Preparation of crystal form II of phosphate

8.5 mg of the compound of formula 1 was dissolved in 0.2 mL of propylene glycol methyl ether to give a clear solution. An aqueous phosphoric acid solution (8.25 µL, 2 M) was added to the resulting solution. The mixture was stirred and centrifuged. The resulting solid was collected and dried under vacuum to obtain a product. Upon detection by X-ray powder diffraction, the product was defined as crystal form II of the phosphate, the XRPD pattern was as shown in FIG. 7, and the positions of the characteristic peaks thereof were as listed in Table 9. The phosphate ion content of the product was detected to be 22.24% by ion chromatography. The DSC pattern shows that the peak temperature of the endothermic peak was 204.80°C. The TGA pattern shows that the weight loss at 30°C-120°C was 0.32%.

**Table 9**

| Peak number | 2θ value [° or degrees] | d[Å] | Relative intensity % |
|---|---|---|---|
| 1 | 7.283 | 12.12888 | 28.6 |
| 2 | 7.659 | 11.53429 | 76.3 |
| 3 | 7.862 | 11.23653 | 76.6 |
| 4 | 8.564 | 10.31644 | 26.1 |
| 5 | 10.049 | 8.79486 | 40.7 |
| 6 | 12.797 | 6.91218 | 34.1 |
| 7 | 13.465 | 6.57082 | 22.5 |
| 8 | 14.303 | 6.18744 | 42.2 |
| 9 | 14.710 | 6.01722 | 39.6 |
| 10 | 15.348 | 5.76864 | 18.7 |
| 11 | 15.921 | 5.56225 | 74.8 |
| 12 | 16.473 | 5.37700 | 18.8 |
| 13 | 17.225 | 5.14393 | 41.7 |
| 14 | 18.373 | 4.82497 | 48.7 |
| 15 | 18.824 | 4.71029 | 67.8 |
| 16 | 19.642 | 4.51606 | 48.5 |
| 17 | 20.380 | 4.35415 | 63.4 |
| 18 | 21.912 | 4.05303 | 16.3 |
| 19 | 23.628 | 3.76244 | 23.1 |
| 20 | 24.682 | 3.60413 | 30.4 |
| 21 | 25.007 | 3.55802 | 11.7 |
| 22 | 25.413 | 3.50203 | 10.2 |
| 23 | 26.357 | 3.37878 | 63.0 |
| 24 | 26.730 | 3.33245 | 100.0 |
| 25 | 27.601 | 3.22917 | 20.3 |

### Example 12: Preparation of crystal form I of methanesulfonate

100 mg of the compound of formula 1 was dissolved in 1 mL of acetone to give a clear solution. An aqueous methanesulfonic acid solution (97.10 µL, 2 M) was added to the resulting solution. The mixture was stirred and centrifuged. The resulting solid was collected and dried under vacuum to obtain a product. Upon detection by X-ray powder diffraction, the product was defined as crystal form I of the methanesulfonate, the XRPD pattern was as shown in FIG. 8, and the positions of the characteristic peaks thereof were as listed in Table 10. The methanesulfonate ion content of the product was detected to be 14.76% by ion chromatography. The DSC pattern shows that the peak temperature of the endothermic peak was 261.30°C. The TGA pattern shows no significant weight loss.

**Table 10**

| Peak number | 2θ value [° or degrees] | d[Å] | Relative intensity % |
|---|---|---|---|
| 1 | 8.311 | 10.62965 | 15.6 |
| 2 | 8.900 | 9.92791 | 4.2 |
| 3 | 9.846 | 8.97621 | 10.0 |
| 4 | 12.785 | 6.91874 | 100.0 |
| 5 | 13.992 | 6.32408 | 11.2 |
| 6 | 14.363 | 6.16190 | 64.1 |
| 7 | 15.220 | 5.81669 | 31.0 |
| 8 | 16.576 | 5.34379 | 13.6 |
| 9 | 17.817 | 4.97439 | 43.0 |
| 10 | 18.248 | 4.85776 | 75.1 |
| 11 | 18.562 | 4.77630 | 6.8 |
| 12 | 19.117 | 4.63883 | 18.2 |
| 13 | 19.318 | 4.59106 | 51.6 |
| 14 | 19.608 | 4.52378 | 68.7 |
| 15 | 20.315 | 4.36782 | 47.4 |
| 16 | 21.075 | 4.21200 | 8.2 |
| 17 | 21.270 | 4.17389 | 17.6 |
| 18 | 21.697 | 4.09268 | 20.0 |
| 19 | 22.256 | 3.99108 | 29.2 |
| 20 | 22.392 | 3.96717 | 21.7 |
| 21 | 22.988 | 3.86576 | 3.8 |
| 22 | 23.221 | 3.82751 | 3.6 |
| 23 | 24.601 | 3.61574 | 3.1 |
| 24 | 24.916 | 3.57078 | 15.1 |
| 25 | 25.404 | 3.50323 | 41.9 |
| 26 | 25.931 | 3.43327 | 17.5 |
| 27 | 26.119 | 3.40893 | 5.6 |
| 28 | 26.936 | 3.30739 | 1.9 |
| 29 | 27.594 | 3.23002 | 19.2 |
| 30 | 27.989 | 3.18531 | 15.3 |
| 31 | 28.411 | 3.13890 | 9.9 |
| 32 | 28.858 | 3.09138 | 26.0 |
| 33 | 29.585 | 3.01698 | 8.3 |
| 34 | 30.347 | 2.94295 | 5.0 |
| 35 | 30.775 | 2.90299 | 2.9 |
| 36 | 31.581 | 2.83070 | 9.9 |
| 37 | 32.111 | 2.78524 | 3.7 |
| 38 | 32.570 | 2.74698 | 2.7 |
| 39 | 33.377 | 2.68242 | 2.7 |
| 40 | 34.062 | 2.63000 | 3.2 |
| 41 | 38.717 | 2.32384 | 3.8 |

### Example 13: Preparation of crystal form II of methanesulfonate

The crystal form I of the methanesulfonate of the compound of formula 1 was stored under conditions of 92.5% RH for 8 days to obtain a product. Upon detection by X-ray powder diffraction, the product was defined as crystal form II of the methanesulfonate, the XRPD pattern was as shown in FIG. 9, and the positions of the characteristic peaks thereof were as listed in Table 11. The methanesulfonate ion content of the product was detected to be 14.60% by ion chromatography. The DSC pattern shows that the peak temperatures of the endothermic peaks were 57.99°C and 262.65°C. The TGA pattern shows that the weight loss at 30°C-120°C was 1.75%.

**Table 11**

| Peak number | 2θ value [° or degrees] | d[Å] | Relative intensity % |
|---|---|---|---|
| 1 | 8.305 | 10.63771 | 100.0 |
| 2 | 9.221 | 9.58340 | 17.3 |
| 3 | 13.430 | 6.58777 | 36.6 |
| 4 | 14.097 | 6.27736 | 15.0 |
| 5 | 15.133 | 5.84997 | 88.5 |
| 6 | 15.631 | 5.66453 | 19.9 |
| 7 | 16.035 | 5.52275 | 11.8 |
| 8 | 16.680 | 5.31059 | 36.0 |
| 9 | 17.223 | 5.14454 | 39.3 |
| 10 | 17.943 | 4.93971 | 22.3 |
| 11 | 18.599 | 4.76697 | 17.8 |
| 12 | 19.130 | 4.63573 | 30.9 |
| 13 | 19.536 | 4.54024 | 41.1 |
| 14 | 20.915 | 4.24385 | 57.6 |
| 15 | 21.185 | 4.19042 | 43.6 |
| 16 | 23.482 | 3.78542 | 37.3 |
| 17 | 25.428 | 3.50004 | 32.2 |
| 18 | 25.997 | 3.42472 | 71.6 |
| 19 | 26.413 | 3.37163 | 20.4 |
| 20 | 26.714 | 3.33441 | 19.6 |
| 21 | 27.351 | 3.25812 | 10.7 |
| 22 | 28.195 | 3.16248 | 13.4 |
| 23 | 30.383 | 2.93952 | 8.3 |

### Example 14: Preparation of crystal form I of tartrate

8.5 mg of the compound of formula 1 was dissolved in 0.2 mL of acetone to give a clear solution. An aqueous tartaric acid solution (16.5 µL, 2 M) was added to the resulting solution. The mixture was stirred and centrifuged. The resulting solid was collected and dried under vacuum to obtain a product. Upon detection by X-ray powder diffraction, the product was defined as crystal form I of the tartrate, the XRPD pattern was as shown in FIG. 10, and the positions of the characteristic peaks thereof were as listed in Table 12. The tartrate ion content of the product was detected to be 23.90% by ion chromatography. The DSC pattern shows that the peak temperatures of the endothermic peaks were 80.16°C and 194.44°C. The TGA pattern shows that the weight loss at 30°C-120°C was 0.73%.

**Table 12**

| Peak number | 2θ value [° or degrees] | d[Å] | Relative intensity % |
|---|---|---|---|
| 1 | 8.150 | 10.84019 | 100.0 |
| 2 | 11.190 | 7.90083 | 17.4 |
| 3 | 12.659 | 6.98698 | 26.1 |
| 4 | 14.003 | 6.31919 | 12.1 |
| 5 | 15.441 | 5.73381 | 46.4 |
| 6 | 16.466 | 5.37936 | 70.5 |
| 7 | 18.005 | 4.92286 | 22.0 |
| 8 | 20.010 | 4.43378 | 33.5 |
| 9 | 20.454 | 4.33852 | 35.4 |
| 10 | 25.087 | 3.54684 | 47.0 |
| 11 | 27.195 | 3.27649 | 21.2 |

### Example 15: Preparation of crystal form I of tartrate

8.5 mg of the compound of formula 1 was dissolved in 0.2 mL of a solvent as shown in Table 13 below to give a clear solution. An aqueous tartaric acid solution (16.5 µL, 2 M) was added to the resulting solution. The mixture was stirred and centrifuged. The resulting solid was collected and dried under vacuum to obtain a product. The product was detected to be the crystal form I of the tartrate by X-ray powder diffraction.

**Table 13**

| Solvent | Crystal form |
|---|---|
| Tetrahydrofuran | Crystal form I of tartrate |
| Propylene glycol methyl ether | Crystal form I of tartrate |

### Example 16: Preparation of crystal form I of p-toluenesulfonate

100 mg of the compound of formula 1 was dissolved in 1 mL of acetone to give a clear solution. An aqueous p-toluenesulfonic acid solution (97.10 µL, 2 M) was added to the resulting solution. The mixture was stirred and centrifuged. The resulting solid was collected and dried under vacuum to obtain a product. Upon detection by X-ray powder diffraction, the product was defined as crystal form I of the p-toluenesulfonate, the XRPD pattern was as shown in FIG. 11, and the positions of the characteristic peaks thereof were as listed in Table 14. The p-toluenesulfonate ion content of the product was detected to be 22.63% by ion chromatography. The DSC pattern shows that the peak temperature of the endothermic peak was 219.72°C. The TGA pattern shows no significant weight loss. DVS test shows that the sample had a weight gain after moisture absorption of about 1.18% under normal storage conditions (i.e., 25°C, 60% RH), about 1.57% under accelerated test conditions (i.e., 70% RH), and about 2.21% under extreme conditions (90% RH). Furthermore, the crystal form was re-detected and found to be unchanged following the DVS test.

**Table 14**

| Peak number | 2θ value [° or degrees] | d[Å] | Relative intensity % |
|---|---|---|---|
| 1 | 6.579 | 13.42474 | 80.5 |
| 2 | 6.708 | 13.16605 | 100.0 |
| 3 | 8.935 | 9.88935 | 21.7 |
| 4 | 9.145 | 9.66261 | 28.7 |
| 5 | 11.199 | 7.89445 | 3.3 |
| 6 | 12.699 | 6.96499 | 11.7 |
| 7 | 13.136 | 6.73467 | 7.8 |
| 8 | 13.450 | 6.57767 | 14.2 |
| 9 | 14.387 | 6.15166 | 3.8 |
| 10 | 15.072 | 5.87327 | 10.2 |
| 11 | 15.275 | 5.79588 | 27.9 |
| 12 | 15.547 | 5.69516 | 10.7 |
| 13 | 15.773 | 5.61395 | 4.9 |
| 14 | 16.672 | 5.31320 | 21.3 |
| 15 | 16.934 | 5.23160 | 31.0 |
| 16 | 17.217 | 5.14629 | 43.5 |
| 17 | 17.389 | 5.09578 | 21.1 |
| 18 | 17.923 | 4.94513 | 8.4 |
| 19 | 18.123 | 4.89092 | 17.3 |
| 20 | 18.821 | 4.71119 | 9.7 |
| 21 | 18.948 | 4.67971 | 19.3 |
| 22 | 19.287 | 4.59843 | 12.6 |
| 23 | 19.468 | 4.55605 | 11.0 |
| 24 | 19.597 | 4.52625 | 4.2 |
| 25 | 20.186 | 4.39559 | 11.5 |
| 26 | 20.971 | 4.23278 | 23.8 |
| 27 | 21.202 | 4.18717 | 5.7 |
| 28 | 21.438 | 4.14150 | 8.2 |
| 29 | 21.616 | 4.10788 | 4.5 |
| 30 | 21.900 | 4.05515 | 6.8 |
| 31 | 22.226 | 3.99645 | 4.0 |
| 32 | 22.499 | 3.94864 | 24.5 |
| 33 | 22.819 | 3.89402 | 9.4 |
| 34 | 23.265 | 3.82024 | 8.1 |
| 35 | 23.831 | 3.73089 | 12.4 |
| 36 | 24.555 | 3.62246 | 4.4 |
| 37 | 24.861 | 3.57860 | 6.8 |
| 38 | 25.636 | 3.47203 | 5.9 |
| 39 | 26.139 | 3.40639 | 10.2 |
| 40 | 26.860 | 3.31654 | 3.4 |
| 41 | 27.055 | 3.29312 | 6.1 |
| 42 | 27.695 | 3.21851 | 5.6 |
| 43 | 27.950 | 3.18967 | 5.4 |
| 44 | 28.845 | 3.09270 | 8.0 |
| 45 | 29.589 | 3.01657 | 11.3 |
| 46 | 31.432 | 2.84377 | 2.6 |
| 47 | 31.870 | 2.80568 | 2.1 |
| 48 | 32.648 | 2.74060 | 2.1 |
| 49 | 33.511 | 2.67197 | 2.0 |
| 50 | 37.005 | 2.42730 | 3.2 |

### Example 17: Preparation of crystal form I of p-toluenesulfonate

8.5 mg of the compound of formula 1 was dissolved in 0.2 mL of tetrahydrofuran to give a clear solution. An aqueous p-toluenesulfonic acid solution (8.25 µL, 2 M) was added to the resulting solution. The mixture was stirred and centrifuged. The resulting solid was collected and dried under vacuum to obtain a product. The product was detected to be the crystal form I of the p-toluenesulfonate by X-ray powder diffraction.

### Example 18: Preparation of crystal form I of p-toluenesulfonate

30 mg of the compound of formula 1 was dissolved in 0.6 mL of solvents as shown in Table 15 below to give a clear solution. An aqueous p-toluenesulfonic acid solution (29.12 µL, 2 M) was added to the resulting solution. The mixture was subjected to dilution crystallization and then centrifuged. The resulting solid was collected and dried under vacuum to obtain a product. The product was detected to be the crystal form I of the p-toluenesulfonate by X-ray powder diffraction.

**Table 15**

| Solvent | Crystal form |
|---|---|
| Acetone : propylene glycol methyl ether (v/v = 1 : 1) | Crystal form I of p-toluenesulfonate |
| Acetone : propylene glycol methyl ether (v/v = 7 : 3) | Crystal form I of p-toluenesulfonate |
| Acetone : n-propanol (v/v = 7 : 3) | Crystal form I of p-toluenesulfonate |

### Example 19: Preparation of crystal form II of p-toluenesulfonate

8.5 mg of the compound of formula 1 was dissolved in 0.2 mL of propylene glycol methyl ether to give a clear solution. An aqueous p-toluenesulfonic acid solution (8.25 µL, 2 M) was added to the resulting solution. The mixture was subjected to dilution crystallization and then centrifuged. The resulting solid was collected and dried under vacuum to obtain a product. Upon detection by X-ray powder diffraction, the product was defined as crystal form II of the p-toluenesulfonate, the XRPD pattern was as shown in FIG. 12, and the positions of the characteristic peaks thereof were as listed in Table 16. The p-toluenesulfonate ion content of the product was detected to be 21.59% by ion chromatography. The DSC pattern shows that the peak temperatures of the endothermic peaks were 168.81°C and 209.30°C. The TGA pattern shows that the weight loss at 30°C-160°C was 10.98%.

**Table 16**

| Peak number | 2θ value [° or degrees] | d[Å] | Relative intensity % |
|---|---|---|---|
| 1 | 7.470 | 11.82476 | 100.0 |
| 2 | 8.564 | 10.31644 | 9.7 |
| 3 | 12.135 | 7.28742 | 28.9 |
| 4 | 13.033 | 6.78754 | 41.2 |
| 5 | 14.508 | 6.10040 | 42.8 |
| 6 | 15.060 | 5.87811 | 48.7 |
| 7 | 16.414 | 5.39620 | 37.0 |
| 8 | 17.415 | 5.08826 | 35.7 |
| 9 | 18.754 | 4.72767 | 49.4 |
| 10 | 19.902 | 4.45760 | 37.0 |
| 11 | 20.505 | 4.32778 | 22.8 |
| 12 | 21.448 | 4.13973 | 15.3 |
| 13 | 22.193 | 4.00228 | 6.9 |
| 14 | 22.819 | 3.89402 | 6.5 |
| 15 | 24.552 | 3.62292 | 65.9 |
| 16 | 25.528 | 3.48647 | 74.1 |
| 17 | 26.561 | 3.35321 | 34.1 |
| 18 | 27.634 | 3.22540 | 29.1 |
| 19 | 28.458 | 3.13390 | 15.7 |
| 20 | 29.227 | 3.05317 | 5.0 |
| 21 | 30.575 | 2.92153 | 22.1 |

### Example 20: Preparation of crystal form I of fumarate

8.5 mg of the compound of formula 1 was dissolved in 0.2 mL of acetone to give a clear solution. Then 1.91 mg of fumaric acid solid was added to the resulting solution. The mixture was stirred and centrifuged. The resulting solid was collected and dried under vacuum to obtain a product. Upon detection by X-ray powder diffraction, the product was defined as crystal form I of the fumarate, the XRPD pattern was as shown in FIG. 13, and the positions of the characteristic peaks thereof were as listed in Table 17. The fumarate ion content of the product was detected to be 20.57% by ion chromatography. The DSC pattern shows that the peak temperature of the endothermic peak was 190.54°C. The TGA pattern shows no significant weight loss.

**Table 17**

| Peak number | 2θ value [° or degrees] | d[Å] | Relative intensity % |
|---|---|---|---|
| 1 | 5.395 | 16.36653 | 97.3 |
| 2 | 7.134 | 12.38130 | 19.6 |
| 3 | 9.564 | 9.23962 | 8.6 |
| 4 | 11.651 | 7.58890 | 43.1 |
| 5 | 11.981 | 7.38112 | 38.6 |
| 6 | 12.669 | 6.98168 | 12.8 |
| 7 | 13.483 | 6.56179 | 24.5 |
| 8 | 13.992 | 6.32419 | 24.0 |
| 9 | 14.222 | 6.22245 | 12.5 |
| 10 | 14.973 | 5.91210 | 29.5 |
| 11 | 16.295 | 5.43512 | 100.0 |
| 12 | 16.893 | 5.24409 | 21.0 |
| 13 | 17.798 | 4.97942 | 65.3 |
| 14 | 19.316 | 4.59159 | 29.9 |
| 15 | 20.818 | 4.26350 | 4.8 |
| 16 | 21.318 | 4.16459 | 6.5 |
| 17 | 21.849 | 4.06448 | 4.6 |
| 18 | 22.420 | 3.96238 | 25.2 |
| 19 | 23.600 | 3.76681 | 16.5 |
| 20 | 24.100 | 3.68976 | 12.7 |
| 21 | 25.194 | 3.53195 | 11.0 |
| 22 | 25.569 | 3.48098 | 12.3 |
| 23 | 26.663 | 3.34065 | 16.1 |
| 24 | 27.471 | 3.24424 | 12.7 |
| 25 | 28.133 | 3.16937 | 5.7 |

### Example 21: Preparation of crystal form I of fumarate

8.5 mg of the compound of formula 1 was dissolved in 0.2 mL of a solvent as shown in Table 18 below to give a clear solution. Then 1.91 mg of fumaric acid solid was added to the resulting solution. The mixture was stirred and centrifuged. The resulting solid was collected and dried under vacuum to obtain a product. The product was detected to be the crystal form I of the fumarate by X-ray powder diffraction.

**Table 18**

| Solvent | Crystal form |
|---|---|
| Tetrahydrofuran | Crystal form I of fumarate |
| Propylene glycol methyl ether | Crystal form I of fumarate |

### Example 22: Stability study under influencing factors

The crystal form I of p-toluenesulfonate was spread out evenly in an open container, and the stability of the sample was investigated under the conditions of illumination (4500 Lux), high temperature (40°C and 60°C) and high humidity (75% RH and 92.5% RH), respectively. The sampling and investigation period was 1 month.

**Table 19 Stability of crystal form I of p-toluenesulfonate under influencing factors**

| **Condition** | **Time (day)** | **Crystal form I of p-toluenesulfonate** | | |
|---|---|---|---|---|
| | | **Colour and trait** | **Purity%** | **Salt form** |
| Initial | 0 | White powder | 99.7 | Crystal form I of p-toluenesulfonate |
| 40°C | 7 days | White powder | 98.7 | Unchanged |
| | 13 days | White powder | 97.6 | Unchanged |
| | 1 month | White powder | 95.2 | Unchanged |
| 60°C | 7 days | White powder | 98.7 | Unchanged |
| | 13 days | White powder | 97.8 | Unchanged |
| | 1 month | White powder | 94.9 | Unchanged |
| 4500 Lux | 7 days | White powder | 99.6 | Unchanged |
| | 13 days | White powder | 99.5 | Unchanged |
| | 1 month | White powder | 99.1 | Unchanged |
| 75% RH | 13 days | White powder | 99.7 | Unchanged |
| | 1 month | White powder | 99.7 | Unchanged |
| 92.5% RH | 13 days | White powder | 99.7 | Unchanged |
| | 1 month | White powder | 99.7 | Unchanged |

| | | | | |
|---|---|---|---|---|
| Conclusions: the crystal form I of p-toluenesulfonate has good physical and chemical stability under high humidity and illumination conditions. | | | | |

### Experimental example 23: Long-term/accelerated stability

The crystal form I of the p-toluenesulfonate was investigated for stability under conditions of 25°C/60% RH and 40°C/75% RH, respectively:

**Table 20 Long-term/accelerated stability of crystal form I of p-toluenesulfonate**

| **Condition** | **Time (day)** | **Crystal form I of p-toluen esulfonate** | | |
|---|---|---|---|---|
| | | **Colour and trait** | **Purity%** | **Salt form** |
| Initial | 0 | White powder | 99.7 | Crystal form I of p-toluenesulfonate |
| 25°C/60% RH | 14 days | White powder | 99.7 | Unchanged |
| | 1 month | White powder | 99.7 | Unchanged |
| | 2 months | White powder | 99.7 | Unchanged |
| | 3 months | White powder | 99.7 | Unchanged |
| | 6 months | White powder | 99.7 | Unchanged |
| | 14 days | White powder | 99.6 | Unchanged |
| | 1 month | White powder | 99.7 | Unchanged |
| 40°C/75% RH | 2 months | White powder | 99.7 | Unchanged |
| | 3 months | White powder | 99.7 | Unchanged |
| | 6 months | White powder | 99.7 | Unchanged |

| | | | | |
|---|---|---|---|---|
| Conclusions: the crystal form I of the p-toluenesulfonate has good physical and chemical stability under long-term/accelerated conditions. | | | | |

## Claims

1. A pharmaceutically acceptable salt of 1-(endo-3-((4-((4-([1,2,4]triazolo[1,5-a]pyridin-7-yloxy)-2-fluoro-3-methylphenyl)amino)quinazolin-6-yl)oxy)-8-azabicyclo[3.2.1]oct-8-yl)prop-2-en-1-one represented by formula 1, **characterised in that** the pharmaceutically acceptable salt is selected from a hydrochloride, a sulfate, a phosphate, a methanesulfonate, a tartrate, a p-toluenesulfonate, and a fumarate,

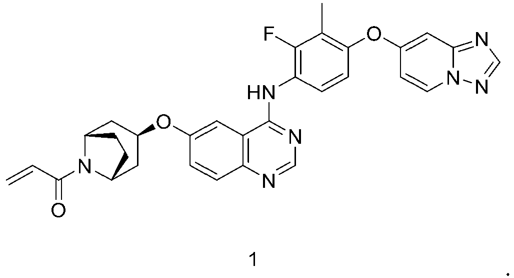

2. The pharmaceutically acceptable salt according to claim 1, **characterised in that** the chemical ratio of 1-(endo-3-((4-((4-([1,2,4]triazolo[1,5-a]pyridin-7-yloxy)-2-fluoro-3-methylphenyl)amino)quinazolin-6-yl)oxy)-8-azabicyclo[3.2.1]oct-8-yl)prop-2-en-1-one to an acid is 3 : 1-1 : 3, preferably 2 : 1-1 : 2, more preferably 1 : 1 or 1 : 2.

3. A method for preparing a pharmaceutically acceptable salt of 1-(endo-3-((4-((4-([1,2,4]triazolo[1,5-a]pyridin-7-yloxy)-2-fluoro-3-methylphenyl)amino)quinazolin-6-yl)oxy)-8-azabicyclo[3.2.1]oct-8-yl)prop-2-en-1-one, **characterised in that** the method comprises a step of subjecting 1-(endo-3-((4-((4-([1,2,4]triazolo[1,5-a]pyridin-7-yloxy)-2-fluoro-3-methylphenyl)amino)quinazolin-6-yl)oxy)-8-azabicyclo[3.2.1]oct-8-yl)prop-2-en-1-one to a reaction with an acid, wherein the acid is selected from hydrochloric acid, sulfuric acid, phosphoric acid, methanesulfonic acid, fumaric acid, p-toluenesulfonic acid and tartaric acid.

4. A methanesulfonate of 1-(endo-3-((4-((4-([1,2,4]triazolo[1,5-a]pyridin-7-yloxy)-2-fluoro-3-methylphenyl)amino)quinazolin-6-yl)oxy)-8-azabicyclo[3.2.1]oct-8-yl)prop-2-en-1-one, **characterised in that** the chemical ratio of 1-(endo-3-((4-((4-([1,2,4]triazolo[1,5-a]pyridin-7-yloxy)-2-fluoro-3-methylphenyl)amino)quinazolin-6-yl)oxy)-8-azabicyclo[3.2.1]oct-8-yl)prop-2-en-1-one to methanesulfonic acid is 1 : 1.

5. A crystal form I of a methanesulfonate of a compound of formula 1, **characterised in that** an X-ray powder diffraction pattern thereof expressed with diffraction angle 2*θ* has characteristic peaks at 12.785, 14.363, 18.248, 19.608, 20.315 and 25.404, preferably has characteristic peaks at 8.311, 12.785, 14.363, 15.220, 17.817, 18.248, 19.318, 19.608, 20.315, 21.697, 22.256, 25.404 and 28.858, and more preferably has characteristic peaks at 8.311, 9.846, 12.785, 14.363, 15.220, 16.576, 17.817, 18.248, 19.318, 19.608, 20.315, 21.697, 22.256, 24.601, 25.404, 27.594, 28.858 and 31.581,

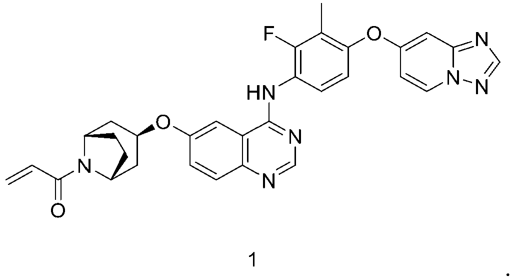

6. The crystal form according to claim 5, **characterised in that** the X-ray powder diffraction pattern expressed with diffraction angle 2*θ* is as shown in FIG. 8.

7. A method for preparing the crystal form according to claim 5 or 6, **characterised in that** the method comprises: dissolving the compound of formula 1 in acetone, adding an aqueous methanesulfonic acid solution, and stirring the mixture.

8. A p-toluenesulfonate of 1-(endo-3-((4-((4-([1,2,4]triazolo[1,5-a]pyridin-7-yloxy)-2-fluoro-3-methylphenyl)amino)quinazolin-6-yl)oxy)-8-azabicyclo[3.2.1]oct-8-yl)prop-2-en-1-one, **characterised in that** the chemical ratio of 1-(endo-3-((4-((4-([1,2,4]triazolo[1,5-a]pyridin-7-yloxy)-2-fluoro-3-methylphenyl)amino)quinazolin-6-yl)oxy)-8-azabicyclo[3.2.1]oct-8-yl)prop-2-en-1-one to p-toluenesulfonic acid is 1 : 1.

9. A crystal form I of a p-toluenesulfonate of a compound of formula 1, **characterised in that** an X-ray powder diffraction pattern thereof expressed with diffraction angle 2*θ* has characteristic peaks at 6.708, 9.145, 15.275, 17.217, 20.971 and 22.499, preferably has characteristic peaks at 6.708, 8.935, 9.145, 13.450, 15.275, 16.934, 17.217, 18.123, 18.948, 20.971, 22.499 and 23.831, and more preferably has characteristic peaks at 6.579, 6.708, 8.935, 9.145, 12.699, 13.450, 15.275, 16.934, 17.217, 18.123, 18.948, 20.186, 20.971, 22.499, 23.831, 26.139 and 29.589.

10. The crystal form according to claim 9, **characterised in that** the X-ray powder diffraction pattern expressed with diffraction angle 2*θ* is as shown in FIG. 11.

11. A method for preparing the crystal form according to claim 9 or 10, **characterised in that** the method comprises: dissolving the compound of formula 1 in solvent V, adding an aqueous p-toluenesulfonic acid solution, and stirring the mixture, wherein the solvent V is selected from one of acetone, tetrahydrofuran, acetone : propylene glycol methyl ether (v/v = 1 : 1), acetone : propylene glycol methyl ether (v/v = 7 : 3), and acetone : n-propanol (v/v = 7 : 3).

12. The crystal form according to any one of claims 5-6 and 9-10, **characterised in that** the error range of the 2*θ* is ±0.20.

13. A pharmaceutical composition, **characterised in that** the pharmaceutical composition comprises the pharmaceutically acceptable salt of 1-(endo-3-((4-((4-([1,2,4]triazolo[1,5-a]pyridin-7-yloxy)-2-fluoro-3-methylphenyl)amino)quinazolin-6-yl)oxy)-8-azabicyclo[3.2.1]oct-8-yl)prop-2-en-1-one according to any one of claims 1, 2, 4 and 8, or the crystal form according to any one of claims 5-6 and 9-10, and optionally a pharmaceutically acceptable excipient.

14. A method for preparing a pharmaceutical composition, **characterised in that** the method comprises a step of mixing the pharmaceutically acceptable salt of 1-(endo-3-((4-((4-([1,2,4]triazolo[1,5-a]pyridin-7-yloxy)-2-fluoro-3-methylphenyl)amino)quinazolin-6-yl)oxy)-8-azabicyclo[3.2.1]oct-8-yl)prop-2-en-1-one according to any one of claims 1, 2, 4 and 8, or the crystal form according to any one of claims 5-6 and 9-10 with a pharmaceutically acceptable excipient.

15. Use of the pharmaceutically acceptable salt of 1-(endo-3-((4-((4-([1,2,4]triazolo[1,5-a]pyridin-7-yloxy)-2-fluoro-3-methylphenyl)amino)quinazolin-6-yl)oxy)-8-azabicyclo[3.2.1]oct-8-yl)prop-2-en-1-one according to any one of claims 1, 2, 4 and 8, or the crystal form according to any one of claims 5-6 and 9-10, or the pharmaceutical composition according to claim 13 in the preparation of a HER2 inhibitor.

16. Use of the pharmaceutically acceptable salt of 1-(endo-3-((4-((4-([1,2,4]triazolo[1,5-a]pyridin-7-yloxy)-2-fluoro-3-methylphenyl)amino)quinazolin-6-yl)oxy)-8-azabicyclo[3.2.1]oct-8-yl)prop-2-en-1-one according to any one of claims 1, 2, 4 and 8, or the crystal form according to any one of claims 5-6 and 9-10, or the pharmaceutical composition according to claim 13 in the preparation of a medicament for the treatment and/or prevention of cancer.
